# EUROPEAN PATENT SPECIFICATION

(11) **EP 3 138 604 B1**
(45) Date of publication and mention of the grant of the patent: **25.11.2020**
(21) Application number: 16184109.3
(22) Date of filing: 26.03.2010
(51) Int. Cl.: A61N 1/36, A61N 1/372, A61N 1/05

(54) **VISUAL PROSTHESIS FITTING SYSTEM**
ANPASSUNGSSYSTEM FÜR SEHPROTHESEN
SYSTÈME POUR L'AJUSTEMENT D'UNE PROTHÈSE VISUELLE

(30) Priority: 27.03.2009 US 164330 P
(43) Date of publication of application: 08.03.2017
(62) Divisional of application: 10722836.3
(73) Proprietor: Second Sight Medical Products, Inc., 12744 San Fernando Road - Suite 400 Sylmar, CA 91342 (US)
(72) Inventor: MCMAHON, Matthew J., DC, WA 20002 (US); GREENBERG, Robert J., Los Angeles, CA 90068 (US); DORN, Jessy, Los Angeles, CA 90026 (US); CASPI, Avraham, I., La Jolla, CA 92037 (US); MCCLURE, Kelly H., Simi Valley, CA 93063 (US)
(74) Representative: Mewburn Ellis LLP

(56) References cited:
- WO-A1-2010/027871
- CN-A- 101 169 683
- US-A1- 2004 172 098
- US-A1- 2004 172 098
- US-A1- 2008 043 201
- US-A1- 2008 043 201
- US-A1- 2008 228 242
- US-A1- 2008 228 242

## Description

### FIELD

The present disclosure relates to visual prostheses configured to provide neutral stimulation for the creation of artificial vision, and more specifically, and improved method of fitting and training for a visual prosthesis.

### BACKGROUND

In 1755 LeRoy passed the discharge of a Leyden jar through the orbit of a man who was blind from cataract and the patient saw "flames passing rapidly downwards." Ever since, there has been a fascination with electrically elicited visual perception. The general concept of electrical stimulation of retinal cells to produce these flashes of light or phosphenes has been known for quite some time. Based on these general principles, some early attempts at devising a prosthesis for aiding the visually impaired have included attaching electrodes to the head or eyelids of patients. While some of these early attempts met with some limited success, these early prosthetic devices were large, bulky and could not produce adequate simulated vision to truly aid the visually impaired.

In the early 1930's, Foerster investigated the effect of electrically stimulating the exposed occipital pole of one cerebral hemisphere. He found that, when a point at the extreme occipital pole was stimulated, the patient perceived a small spot of light directly in front and motionless (a phosphene). Subsequently, Brindley and Lewin (1968) thoroughly studied electrical stimulation of the human occipital (visual) cortex. By varying the stimulation parameters, these investigators described in detail the location of the phosphenes produced relative to the specific region of the occipital cortex stimulated. These experiments demonstrated: (1) the consistent shape and position of phosphenes; (2) that increased stimulation pulse duration made phosphenes brighter; and (3) that there was no detectable interaction between neighboring electrodes which were as close as 2.4 mm apart.

As intraocular surgical techniques have advanced, it has become possible to apply stimulation on small groups and even on individual retinal cells to generate focused phosphenes through devices implanted within the eye itself. This has sparked renewed interest in developing methods and apparatuses to aid the visually impaired. Specifically, great effort has been expended in the area of intraocular visual prosthesis devices in an effort to restore vision in cases where blindness is caused by photoreceptor degenerative retinal diseases such as retinitis pigmentosa and age related macular degeneration which affect millions of people worldwide.

Neural tissue can be artificially stimulated and activated by prosthetic devices that pass pulses of electrical current through electrodes on such a device. The passage of current causes changes in electrical potentials across visual neuronal membranes, which can initiate visual neuron action potentials, which are the means of information transfer in the nervous system.

Based on this mechanism, it is possible to input information into the nervous system by coding the information as a sequence of electrical pulses which are relayed to the nervous system via the prosthetic device. In this way, it is possible to provide artificial sensations including vision.

One typical application of neural tissue stimulation is in the rehabilitation of the blind. Some forms of blindness involve selective loss of the light sensitive transducers of the retina. Other retinal neurons remain viable, however, and may be activated in the manner described above by placement of a prosthetic electrode device on the inner (toward the vitreous) retinal surface (epiretial). This placement must be mechanically stable, minimize the distance between the device electrodes and the visual neurons, and avoid undue compression of the visual neurons.

In 1986, Bullara (US Pat. No. 4,573,481) patented an electrode assembly for surgical implantation on a nerve. The matrix was silicone with embedded iridium electrodes. The assembly fit around a nerve to stimulate it.

Dawson and Radtke stimulated cat's retina by direct electrical stimulation of the retinal ganglion cell layer. These experimenters placed nine and then fourteen electrodes upon the inner retinal layer (i.e., primarily the ganglion cell layer) of two cats. Their experiments suggested that electrical stimulation of the retina with 30 to 100 uA current resulted in visual cortical responses. These experiments were carried out with needle-shaped electrodes that penetrated the surface of the retina (see also US Pat. No. 4,628,933 to Michelson).

The Michelson '933 apparatus includes an array of photosensitive devices on its surface that are connected to a plurality of electrodes positioned on the opposite surface of the device to stimulate the retina. These electrodes are disposed to form an array similar to a "bed of nails" having conductors which impinge directly on the retina to stimulate the retinal cells. US Patents 4,837,049 to Byers describes spike electrodes for neural stimulation. Each spike electrode pierces neural tissue for better electrical contact. US Patent 5,215,088 to Norman describes an array of spike electrodes for cortical stimulation. Each spike pierces cortical tissue for better electrical contact.

The art of implanting an intraocular prosthetic device to electrically stimulate the retina was advanced with the introduction of retinal tacks in retinal surgery. De Juan, et al. at Duke University Eye Center inserted retinal tacks into retinas in an effort to reattach retinas that had detached from the underlying choroid, which is the source of blood supply for the outer retina and thus the photoreceptors. See, e.g., E. de Juan, et al., 99 Am. J. Ophthalmol. 272 (1985). These retinal tacks have proved to be biocompatible and remain embedded in the retina, and choroid/sclera, effectively pinning the retina against the choroid and the posterior aspects of the globe. Retinal tacks are one way to attach a retinal array to the retina. US Patent 5,109,844 to de Juan describes a flat electrode array placed against the retina for visual stimulation. US Patent 5,935,155 to Humayun describes a visual prosthesis for use with the flat retinal array described in de Juan.

In an implantable visual prosthesis system, the array attached to the retina is very rarely centered and perfectly oriented about the center of the visual field. When an implanted individual's eyes are at a neutral forward looking and level gaze, this is where the brain expects to see the image created by the electrical stimulation of the array. If the camera mounted on the subject's head is looking forward and level and the array is superior to the preferred location, this lack of correspondence between the real world scene and the location of perception will result in the image from the scene in the center of the camera image appearing to the subject as being inferior, which can cause confusion in the down stream visual processing systems such as the LGN (thalamus), superior colliculus, and visual cortex. Similar issues arise with temporal and nasal misalignment as well as rotation.

Further, imperfect position of the camera itself relative to the subject's head can cause the same problem.

### SUMMARY

The present invention is an improved fitting and training system for a visual prosthesis. A patient, using the visual prosthesis observes a display and indicates location, movement, shape or other properties of the display image to provide for improved fitting and training. In one embodiment, the patient uses a touch sensitive monitor which displays an image. The patient touches the monitor at the location where the patient perceives the image. The system then corrects the image to the location indicated by the patient. In another embodiment a patient observes an image moving across the touch sensitive monitor and indicates by moving their hand across the monitor which direction the patient believes the image is moving. The system can then rotate the image to match the image perceived by the patient. These corrections may be automated by input from the touch sensitive monitor or controlled by a clinician.

Further embodiments are shown in the specification, drawings and claims of the present application.

### BRIEF DESCRIPTION OF THE FIGURES

**FIGS. 1** A - R are a series of scatter plots showing patients' performance using square localization.
**FIGS. 2** **A and B** are a series of bar graphs showing accuracy versus distance.
**FIGS. 3** **A - R** are a series of bar graphs showing accuracy versus number of trials.
**FIG. 4** is a table showing accuracy with the device off or on.
**FIG. 5** is a table showing accuracy with the device off or on.
**FIGS. 6** **and** **7** show a video capture/transmission apparatus or visor adapted to be used in combination with the retinal stimulation of Figures 16 and 17.
**FIG 8** shows a flexible circuit electrode array.
**FIG 9** shows components of a fitting system according to the present disclosure, the system also comprising the visor shown in Figures 6 and 7.
**FIG 10** shows the external portion of the visual prosthesis apparatus in a stand-alone mode, i.e.
comprising the visor connected to a video processing unit.
**FIGS 11-12** show the video processing unit in more detial already briefly shown with reference to Figure 9 and 10.
**FIG 13a** shows a LOSS OF SYNC mode.
**FIG 13b** shows an exemplary block diagram of the steps taken when VPU does not receive back telemetry from the Retinal stimulation system.
**FIG 13c** shows an exemplary block diagram of the steps taken when the subject is not wearing Glasses.
**FIG 14-1****,** 14-2, 14-3 and 14-4 show an exemplary embodiment of a video processing unit. FIG 14-1 should be viewed at the left of Figure 14-2 . Figure 14-3 should be viewed at the left of Figure 14-4 . Figures 14-1 and 14-2 should be viewed on top of Figures 14-3 and 14-4.
**FIG. 15** is flow chart of the video processing chain in a visual prosthesis
**FIG. 16** is a perspective view of the implanted portion of the preferred visual prosthesis.
**FIG. 17** is a side view of the implanted portion of the preferred visual prosthesis showing the fan tail in more detail.
**FIG. 18** is a view of the completed package attached to an electrode array.
**FIG. 19** is a cross-section of the package.

### DETAILED DESCRIPTION

The present invention is an improved fitting and training system for a visual prosthesis. In the preferred embodiment described below, the patient uses a touch sensitive monitor which displays an image. The patient touches the monitor at the location where the patient perceives the image. The system then corrects the location of the image, as seen through the visual prosthesis, to the location indicated by the patient. In another preferred embodiment a patient observes an image moving across the touch sensitive monitor and indicates by moving their hand across the monitor which direction the patient believes the image is moving. The system can then rotate the image, as seen through the visual prosthesis, to match the image perceived by the patient to the image display by the touch screen. These corrections may be automated by input from the touch sensitive monitor or controlled by a clinician. This correction to the image presented to the patient by the visual prosthesis fits the prosthesis to the patient so later observations of the real world, as seen through the visual prosthesis, more accurately represent the world observed by the patent.

### Square or Circle Localization:

In the Square Localization test, a high-contrast white square (200 x 200 pixels) was presented in random locations on a 20" touch screen monitor in front of the subject. When prompted, the subject scanned the monitor and located the square, touching the screen at the location of the square center. Subjects first completed a short practice run (10-trial) in training mode, in which they selected the location of the square by touching the monitor where they wanted it to appear. Next, a 40-trial test was administered. No feedback was given to the subject during the test.

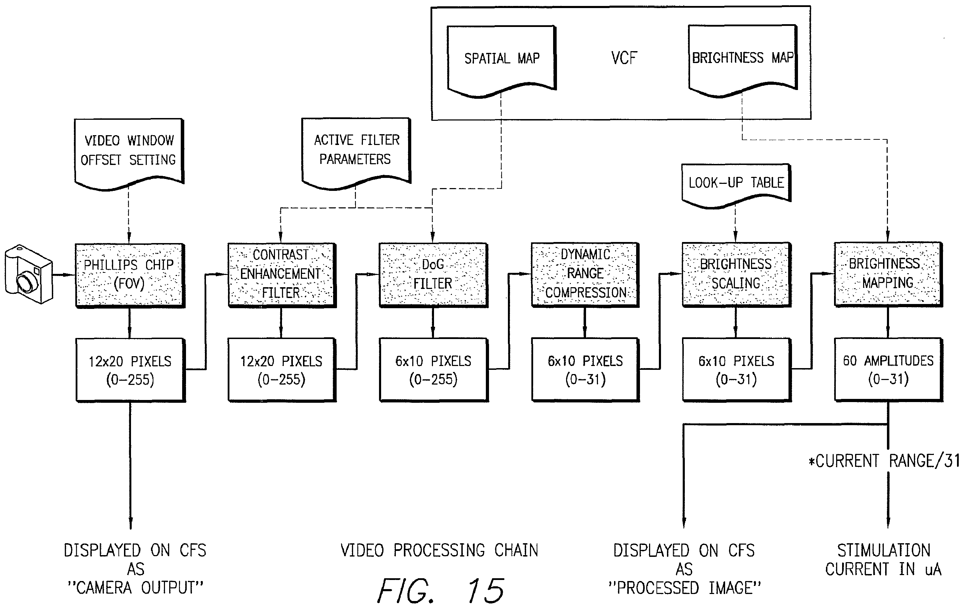

The mean and standard deviation from the square localization assessment can be used to adjust the field of view of the camera. This can be done by physically moving the camera angle with respect to the glasses or translating the camera position with respect to the glasses. This can also be done electronically by selecting the appropriate field of view from the camera signal to feed to the implant. Because the image captured by the camera and stored in memory is much larger than the field of view associated with the electrode array on the retina, electronic control can be accomplished by down-selecting an appropriate window of video data from the image. Refer to "Video Window offset Setting" with respect to Figure 15.

Further, another shape such as circle or square with an intensity that is brightest at the center that gradual fades out may reduce edge effects and measurement error. The size of the shape could be set to the minimum the size that is detectable by the subject, further reducing the measurement error.

The x (horizontal) and y (vertical) location of the mean value of the square localization assessment gives you the apparent location that the patient believes the center of mass of the perceptions are appearing. By correcting for this offset with camera position or field of view position, the patient's perceptions can be aligned with visual stimuli from the monitor. For example, if the mean value was 1 cm to the right of the expected center location, the camera could be angled to the right by 1cm (or the field of interest could be electronically moved to the right by 1 cm). This, of course, works in the vertical direction too.

Note that the utility of mean, variance and other statistical measures is largely due to the error in measurement that comes from the nature of the task. It has been noted elsewhere that even sighted subjects have a large degree in of error in tasks of this type when they are not able to see their hand as they proceed to point to a target. There are other analyses besides mean and standard deviation that may be more advantageous. For instance, cluster analysis of the data would be helpful when the points fall into more than one region. A moment analysis (center of mass) approach where the pixels were weighted would also improve the precision of the point.

Further, it is also likely that the sources of error sum to locations with a probability density function that is not Gaussian, but rather could have several modes, and it is unlikely that the statistics of the random variables that characterize the measurement error are stationary within an electrode and ergodic over the ensemble of all of the electrodes. Thus non Gaussian distributions can be used to estimate the location of perception. This can include but is not limited to Binomial, Poison with parameter, Geometric with parameter, negative binomial with parameters, and Gamma with parameters. These distribution functions are known in the context of estimation, stochastics, and the characterization or random variables. In the case where the measurement errors are mixed, there will be a central location that is close to the true measurements, and overlapping data from probability distributions of the various error sources. In this case, minimum error classification can be used to select the most likely target to use when adjusting the camera. There are several types of appropriate minimum error classification methods that are known in the art.

A variant on the method discussed above is to make many measurements at one stimulation location and then use the estimated location to physically or electronically move the camera toward this location. This could be done with several other stimulation locations until the difference between the stimulation location and the presented square converge to within an acceptable limit.

In another embodiment, the sum of the errors over all electrodes could be used to set the convergence criterion.

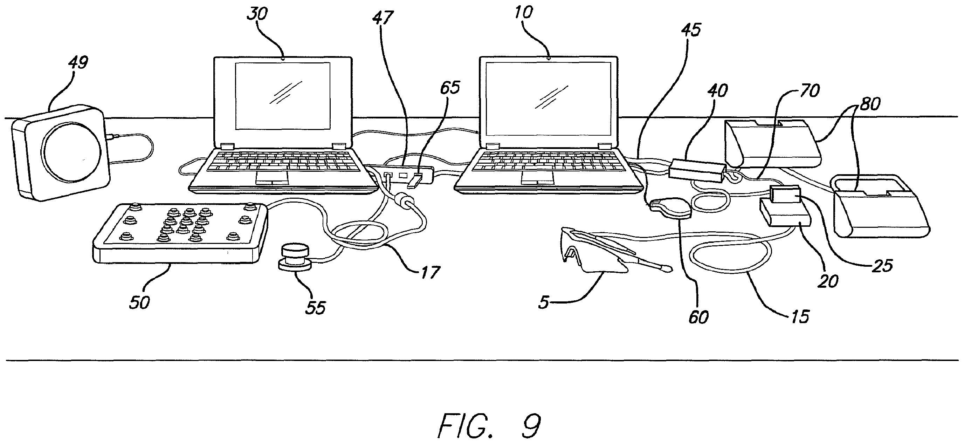

In another embodiment, using the preferred fitting system as shown in figure 9, an image may be adjusted manually by the following steps.
1. Be sure the touch screen monitor is connected to the patient testing system (PTS) computer and set to be the primary monitor.
2. Adjust the height of touch screen monitor so that the camera is pointed to the center of the touch screen.
3. Open the fitting sofware on the PTS laptop, change the directory to the folder containing "Camera Alignment" vl .00, type "runCameraAlignment" and hit Enter. Click on "Camera Position" button. A blank screen will appear on the touch screen.
4. Log in to the clinical fitting system (CFS) and select the "Psychophysics" tab. Log on to PTS and select the "Direct Stimulation" button. Make sure the subject's video processing unit (VPU) is on and connected to CFS and the subject is wearing the glasses.
5. In the PTS "Direct Stimulation" Screen, stimulate a small group of electrodes in the center of the array, and increase the stimulation amplitude and the number of stimulating electrodes until the subject clearly sees localized bright phosphenes.
6. Adjust the subject seating position and the touch screen monitor in order to align the camera to the center of the touch screen and about 12" away from the screen. Instruct the subject to look straight ahead while keeping their head position as still as possible. Use a chin rest if necessary. Generate a phosphene using Direct Stimulation and ask the subject to point the location of the phosphene on the touch screen without moving their eyes or their head. If the position of the phosphene is not on the touch screen, move the touch screen or adjust the height of the subject's chair so that the response is on the monitor. Verify that a gray symbol appears on the touch screen at the location indicated by the subject.
7. Repeat the stimulation and gather a response 8 times. The touch screen will display all the outputs from the subject. Click the "Undo last trial" button to remove the last responses from the subject if necessary. Click the "Back" button to go back to the main screen and click the "Exit" button to exit the program. If the touch screen monitor or the subject seating is adjusted during this step, repeat the step to collect 8 responses.
8. The program will calculate the average position of the responses and present an alignment target (a white circle) centered at this position on the touch screen.
9. Log out of PTS. Select the "CamPos" tab in CFS. Instruct the subject to look straight ahead and to carefully maintain the same head and body position as during the data collection phase. The alignment target should appear in "CamPos" screen. If not, use the right arrow key on the PTS to increase the size of the alignment target until it appears on the "CamPos" screen. Adjust the top, bottom, left, and right arrows on the CFS screen until the alignment target on the touch screen appears at the center of the "CamPos" screen. Reduce the alignment target size if necessary by pressing the left arrow key on the PTS. Click the "save" button on the CFS "CamPos" screen when the alignment target is at the center of the screen. This will select and store the section of the camera image that is aligned with the implant's visual field position on the subject's VPU. Record the saved camera position in the CRF.
10. Run the Square Localization test again to compare with the baseline data.

The ellipse from the square localization assessment is also useful in setting up the field of view of the camera. The area of the ellipse might be used to adjust the zoom of the camera - ie. one might 'zoom out' for a large ellipse or 'zoom in' for a small ellipse. Also, the orientation of the ellipse could be used to adjust the angle or tilt of the camera or field of view. Finally, after adjusting for angle, if the ellipse is not a circle, the ellipse could be used to adjust the horizontal and vertical zoom independently. So, if the ellipse was longer horizontally, a larger horizontal field of view compared to the vertical field of view could be selected.

One additional advantage of the approach is for setting the camera position / field of view is that the entire process can be automated. Thus, a patient can sit in front of a screen that presents individual square stimuli. The patient then touches the touch screen where he/she believes they saw the spot of light. This is repeated for an entire set of locations. The data is then analyzed in real-time (automatically) as described above and automatically downloaded to the VPU to adjust the camera field of view in real-time. In fact, this can be done during the course of the experiment such that data is taken, the field of view adjusted and more data is taken to confirm that the alignment was completed successfully.

Similarly, the direction of motion software can be used to adjust and confirm camera / field of view angle with respect to the horizon. The camera can be rotated (physically or electronically) in real-time until the number of correct responses at zero degrees is maximized. Alternately, the area under the response curve can be integrated and the mean value calculated such that the angle which minimizes the mean value is chosen.

Two spatial vision tests have been developed to supplement Grating Visual Acuity, our primary clinical trial endpoint. These assessment tools, the Square Localization and Direction of Motion tests in the Argus Training Program, were developed to provide an objective measure of spatial vision in subjects who do not reach the lowest levels of the Grating Acuity scale (2.9 logMAR), but who still receive useful spatial information by using the Argus II system. The Square Localization and Direction of Motion tests, device ON and device OFF, were administered to all US Argus II subjects who had been implanted at least 6 months ago (see below for the sole exception).

### Research Results - Square Localization

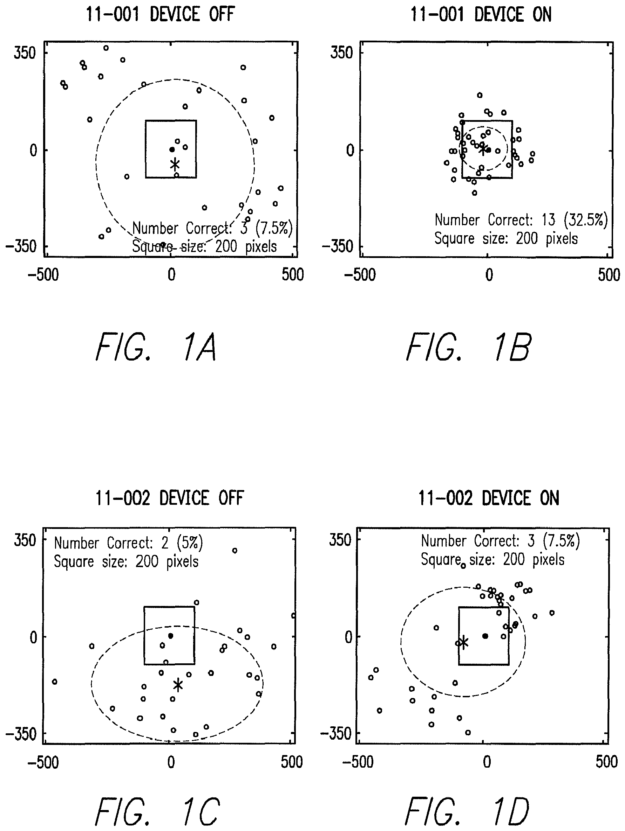

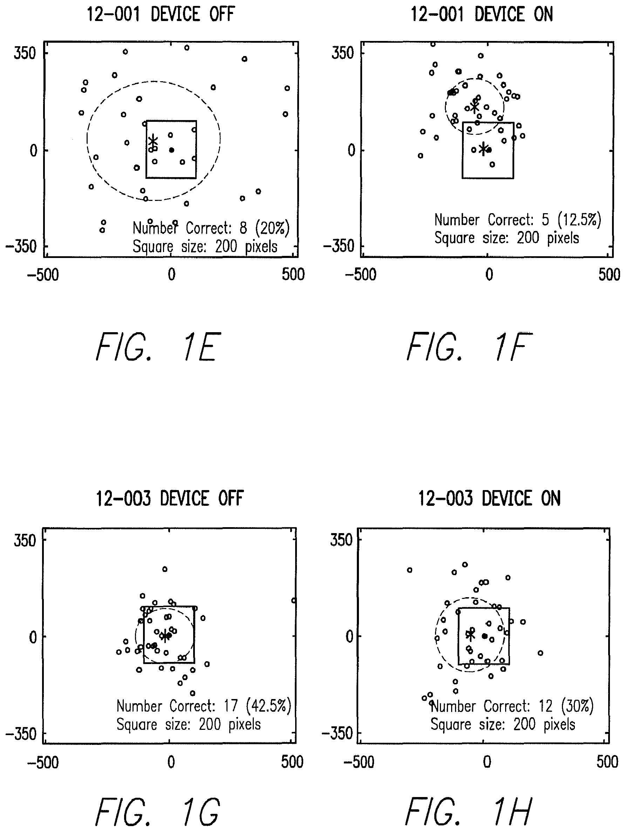

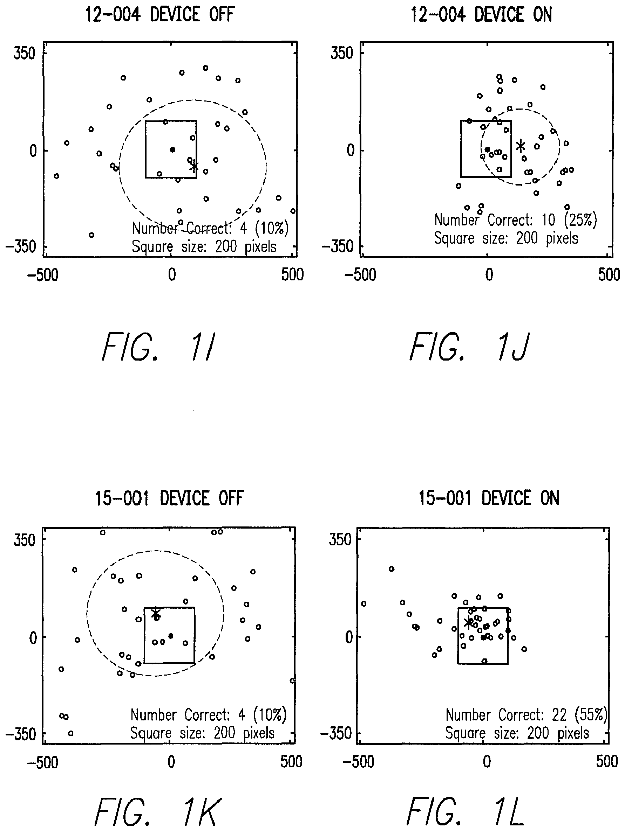

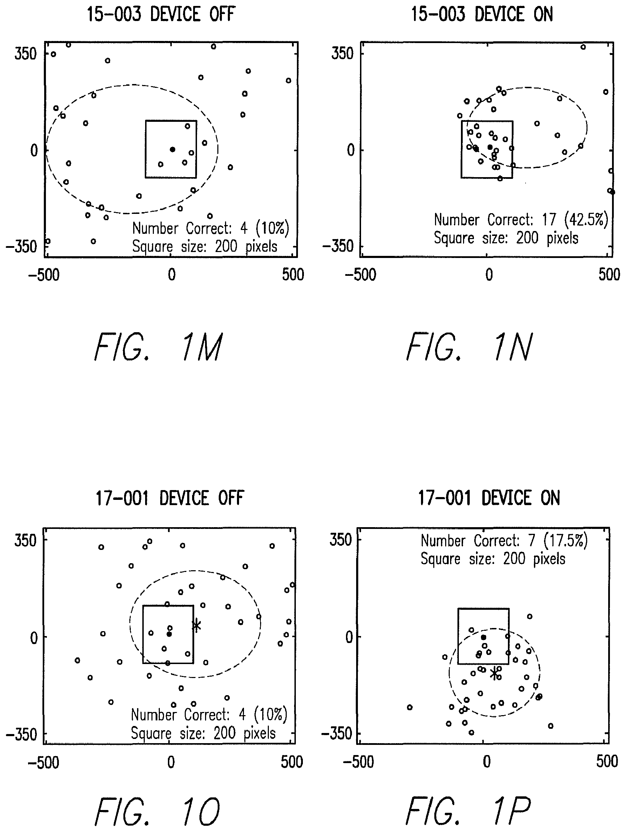

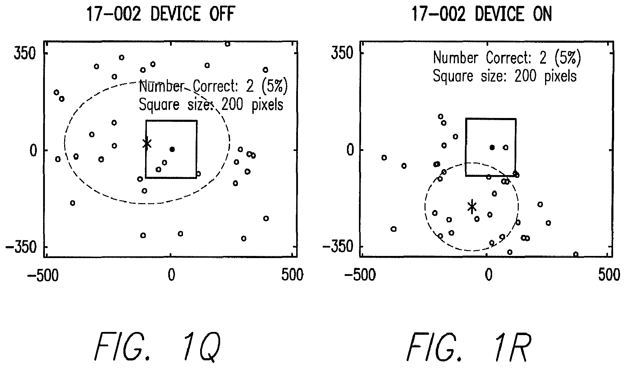

Figure 1 compares device OFF (left) and device ON (right) performance for each subject. Each graph is scaled to the dimensions of the touch screen monitor (1024 x 768 pixels) centered at 0,0.

The subject's response in each trial (open circles) is plotted relative to the center of the square (closed circle), normalized to (0,0). The mean of the responses is plotted as an asterisk, and the standard deviation of the responses is plotted as a dashed ellipse. The number of correct responses (responses within the borders of the square) is also shown on each graph.

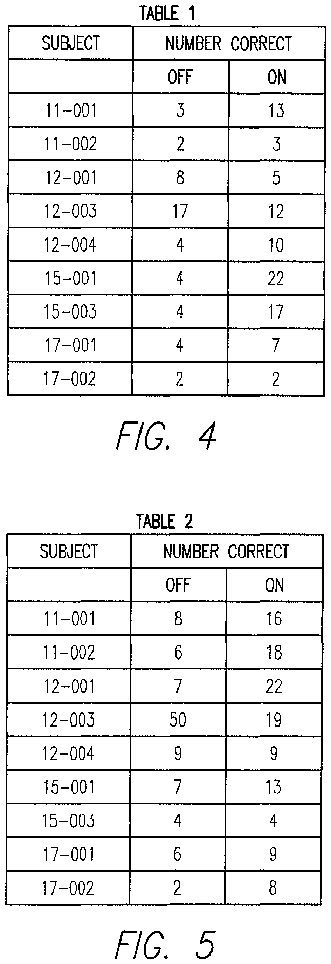

A summary of the number correct for each subject in each condition is provided in Table 1 (**Figure 4**).

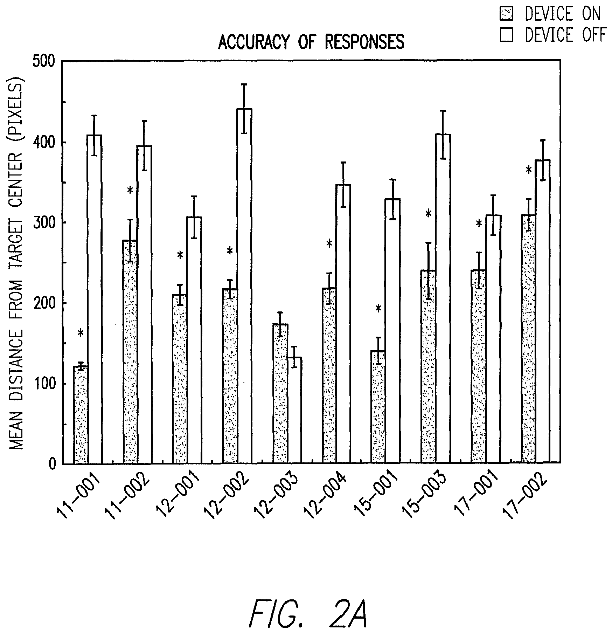

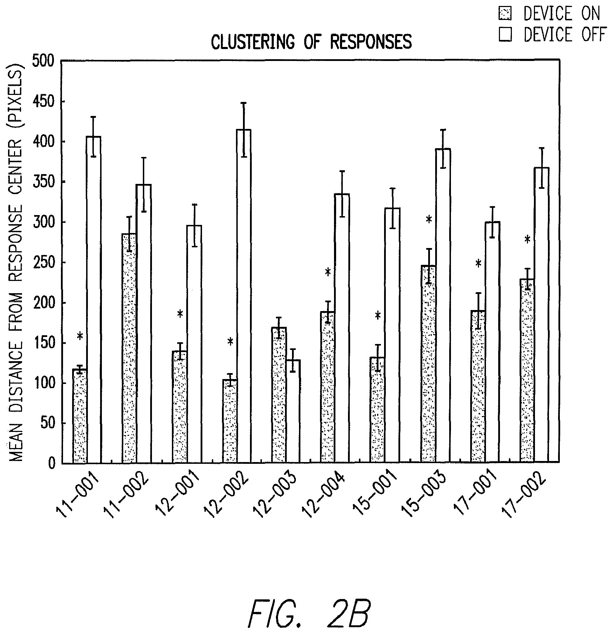

To determine whether device OFF results were significantly different than device ON results, we considered two measures: accuracy, how close the responses were to the center of the target, and clustering, how close the responses were to the center (mean) of the responses. To measure accuracy, we calculated the distance of each response from the center of the target in pixels for both OFF and ON conditions. A two-tailed Welch's t-test (assuming unequal variances) was then used to determine whether the means were likely to have come from the same distribution (null hypothesis: the means of the OFF and ON distributions are equal). Similarly, to measure the extent of clustering, we calculated the distance of each response from the center (mean) of the responses in OFF and ON conditions and tested the null hypothesis that the means were equal. Figure 2 below shows the mean response distance from the center of the target (**Fig. 2-A**) or center of the responses (**Fig. 2-B**) with standard error bars. Statistically different means are indicated with a star (null hypothesis was rejected, p<0.05).

### Research Conclusions - Square Localization

In all but one case, the device ON condition resulted in greater accuracy in the Square Localization task than device OFF; the mean response distance from the center of the target was significantly different in OFF and ON conditions for all subjects except 12-003. In addition, most subjects had more tightly clustered responses in device ON than device OFF; only 11-002 and 12-003's ON responses were not significantly more clustered than the OFF.

In some cases, such as 11-001 and 15-001, the difference between the conditions was very dramatic. 11-001 got 3 correct among many highly-spread responses with device OFF, and 13 correct among responses that were tightly clustered around the square. 15-001 improved from 4 correct with device OFF to 22 correct with device ON.

Several other subjects' performances suggest that their camera alignment should be adjusted - 12-001's responses in device ON, for example, were clustered primarily above the target square, while 17-001's were clustered mostly to the bottom right. The new software release A2E6 should allow us to use the information from these experiments to select a better aligned section of the video image.

One subject, 12-003, had better performance in device OFF than ON, though the difference was not statistically significant for either accuracy or clustering in a two-tailed test. Based on this result and subjective analysis of his residual vision, it seems likely that this subject's vision without the device is better than expected.

### Direction of Motion

In the Direction of Motion test, a high-contrast white bar sweeps across a black background on the monitor. The bar's angle was randomly chosen from all 360° (in 1° increments); the bar moved in a direction orthogonal to its orientation angle (for example, a vertical bar moved left to right across the screen). The subjects' task is to fixate the camera on the center of the monitor and allow the bar to sweep across the field of view. They then drew the direction of motion of the bar on the touch screen monitor. After a short training run, an 80-trial test was administered; no feedback was give to the subject during the test. The speed and width of the bar were fixed for all subjects.

Both tests were administered in both device ON and device OFF conditions; for device OFF, the subjects' eyes were both open and they were not wearing their Argus II glasses. For device ON, both eyes were open, but the subject wore their glasses; room lights were off in both conditions. All US subjects were tested between 5/13/08 and 5/30/08 except 12-002, who was not tested until 8/08 due to health issues, and 15-003, who was tested in 8/08 when she had been implanted for 4 months.

### Research Results - Direction of Motion

The following figures show the Direction of Motion results for each subject, device OFF (left) and ON (right). For each trial, the angle difference between the direction of the target bar and the subject's response was calculated; positive angle differences correspond to a counter-clockwise direction, while negative angle differences correspond to a clockwise direction. The angle differences of all 80 trials are shown in a histogram. A star between the device OFF and device ON graphs indicates that the distributions were found to be significantly different (p<0.05) with a two-sample Kolmogorov-Smirnov test (null hypothesis: the two distributions are equal).

Correct responses for each condition are summarized in Table 2 (**figure 5**); responses were deemed "correct" if the angle difference was +/- 15° from the target direction.

### Research Conclusions - Direction of Motion

It is clear that some subjects are more accurate in judging direction of motion with device on than off. The non-parametric Kolmogorov-Smirnov test found that the device OFF and ON distributions were significantly different in four cases: 11-001, 11-002, 12-001 and 12-003. Indeed, results from three of those subjects (both site 11 subjects and 12-001) show the pattern that we might expect to see - evenly/randomly distributed responses with device OFF, but a distribution that peaks around 0° angle difference with device ON. All three of these subjects also had more correct responses in the ON condition. Subjects 12-004, 15-001, 15-003, 17-001 and 17 -002 do not have significantly different results with device ON than OFF. And subject 12-003, again, showed better results with device OFF than ON; the distributions were significantly different according to the K-S test. This is likely due to a higher level of residual vision than we had previously appreciated.

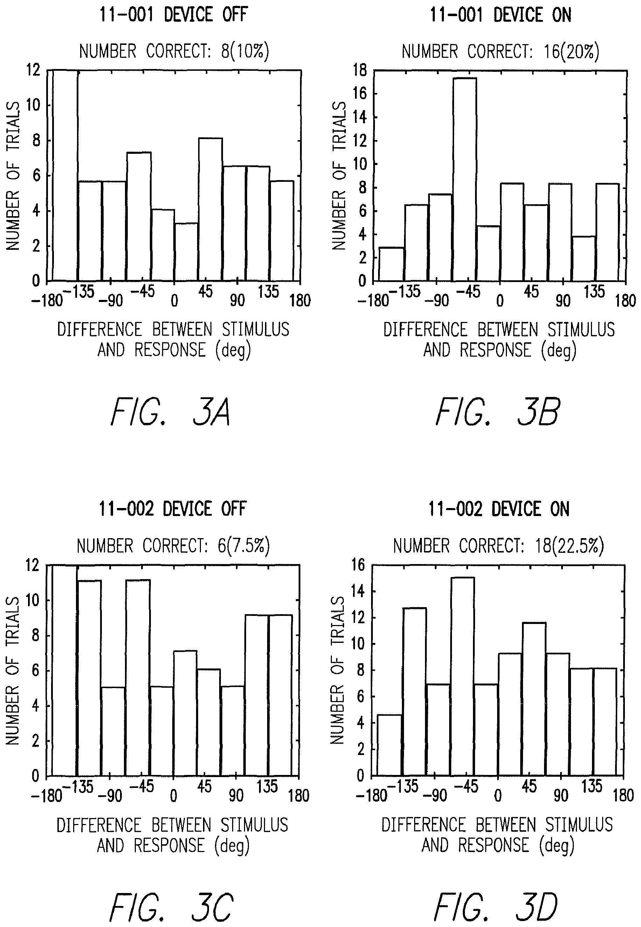

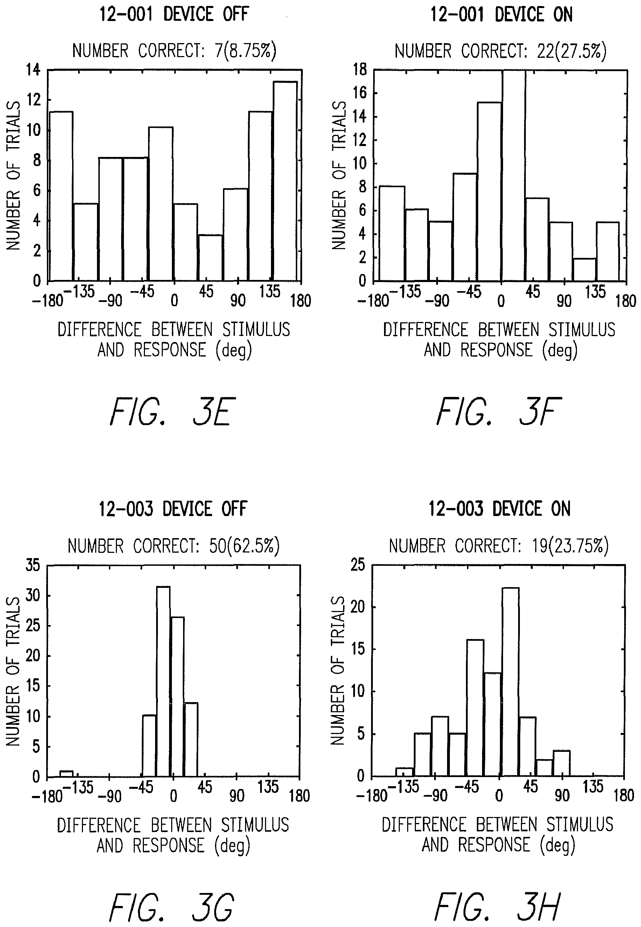

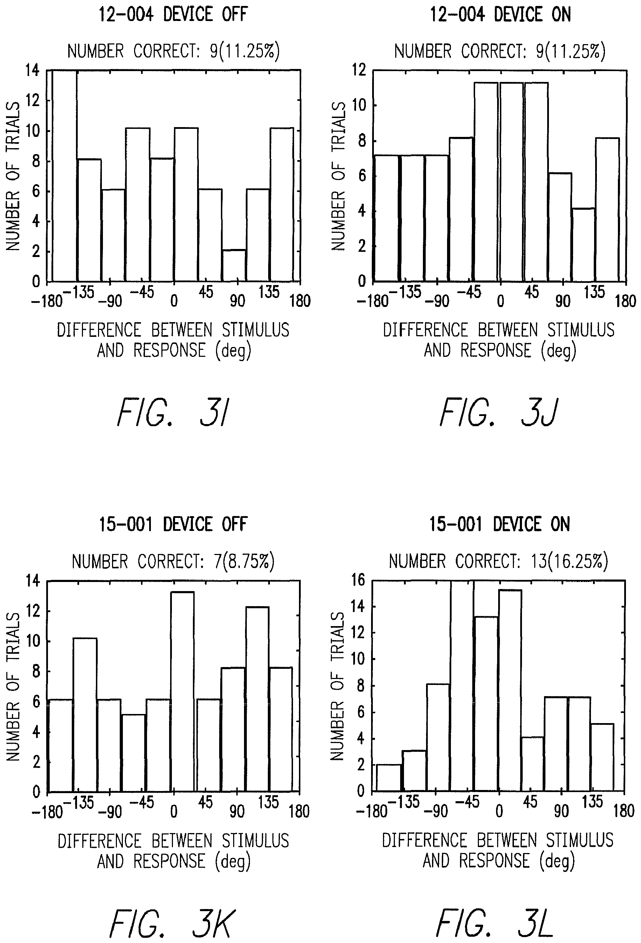

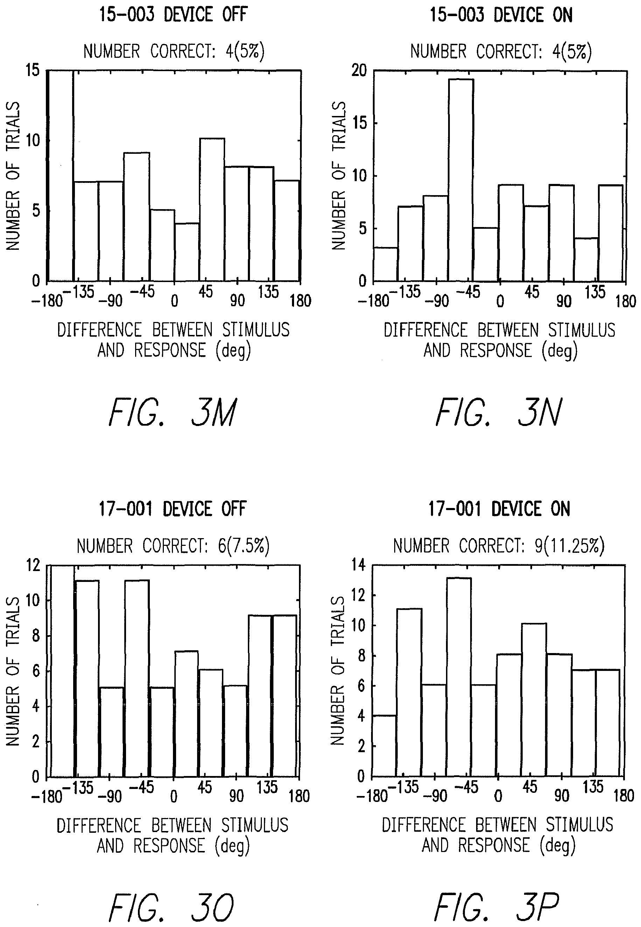

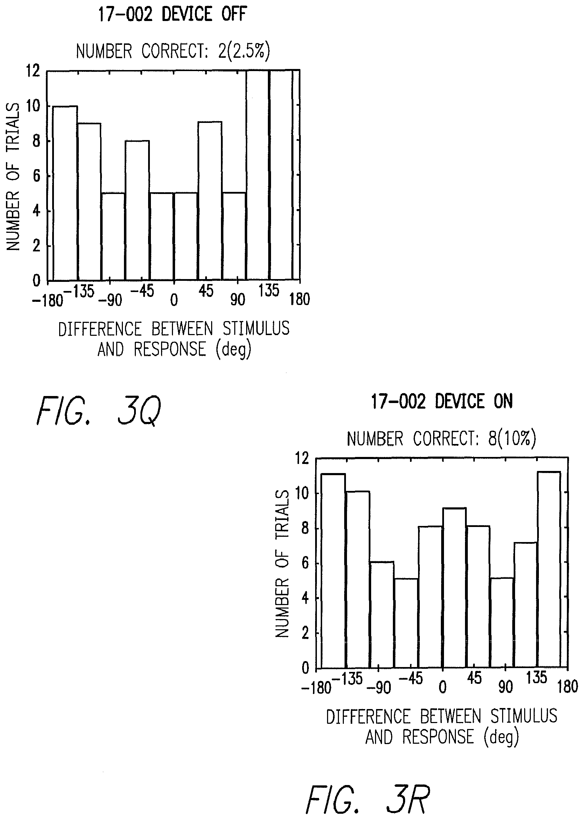

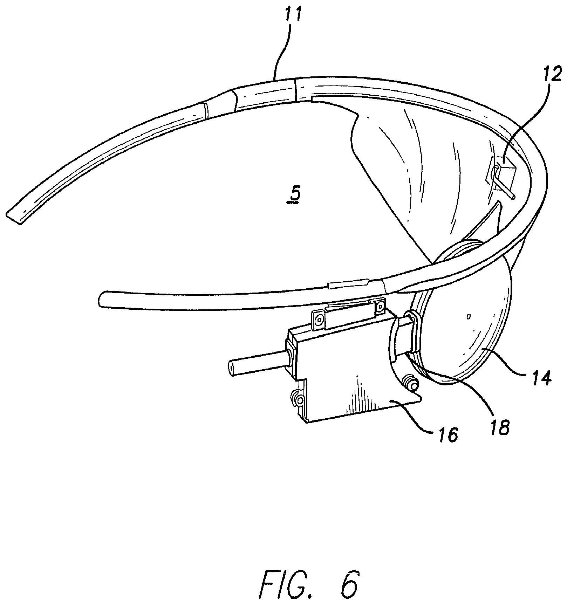

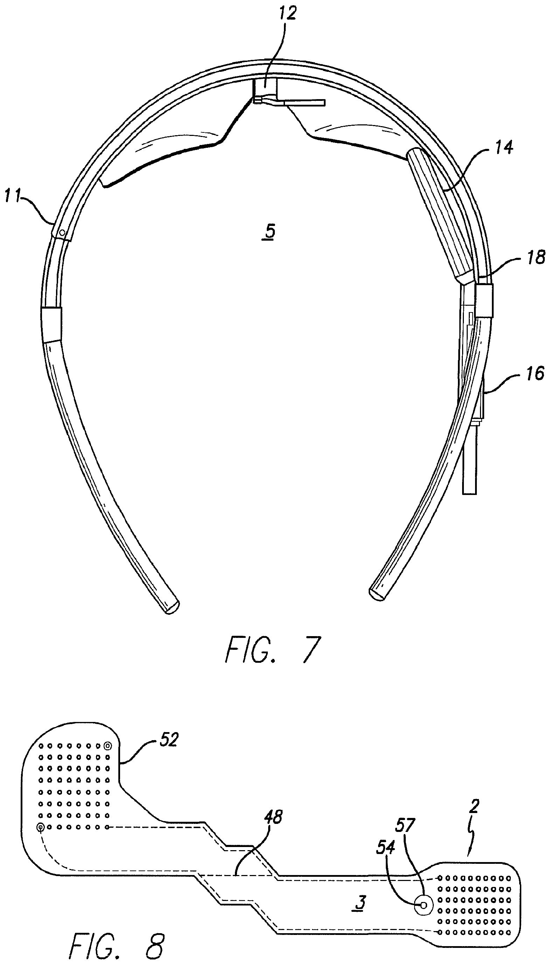

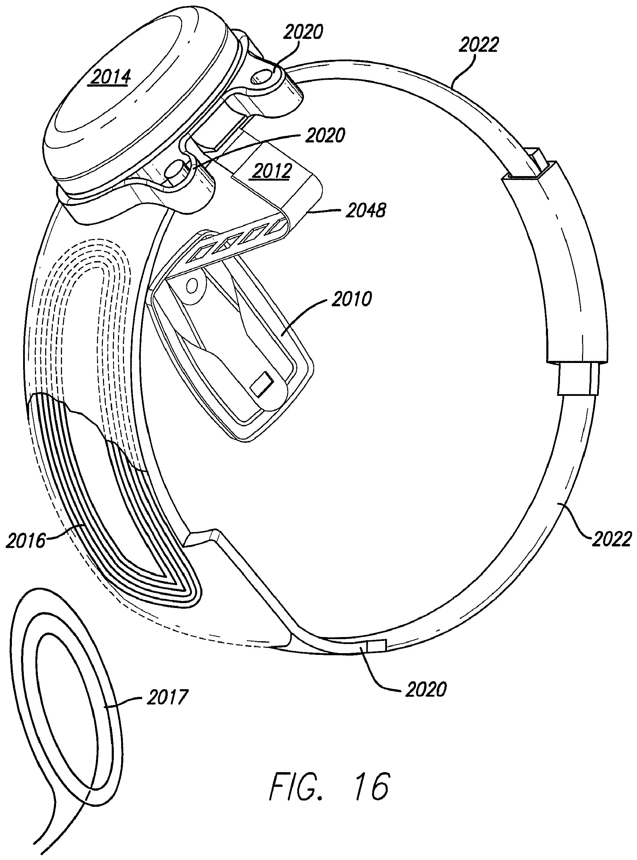

Referring to **Figures 6** and **7**, the glasses **5** may comprise, for example, a frame **11** holding a camera **12**, an external coil **14** and a mounting system **16** for the external coil **14**. The mounting system **16** may also enclose the RF circuitry. In this configuration, the video camera **12** captures live video. The video signal is sent to an external Video Processing Unit (VPU) **20** (shown in **Figures 9****,** **11** and **12** and discussed below), which processes the video signal and subsequently transforms the processed video signal into electrical stimulation patterns or data. The electrical stimulation data are then sent to the external coil **14** that sends both data and power via radio-frequency (RF) telemetry to the coil **116** of the retinal stimulation system **1**, shown in **Figures 16** and **3**. The coil **116** receives the RF commands which control the application specific integrated circuit (ASIC) which in turn delivers stimulation to the retina of the subject via a thin film electrode array (TFEA). In one aspect of an embodiment, light amplitude is recorded by the camera **12**. The VPU **20** may use a logarithmic encoding scheme to convert the incoming light amplitudes into the electrical stimulation patterns or data. These electrical stimulation patterns or data may then be passed on to the Retinal Stimulation System **1,** which results in the retinal cells being stimulated via the electrodes in the electrode array **2** (shown in **Figure 16**). In one exemplary embodiment, the electrical stimulation patterns or data being transmitted by the external coil **14** is binary data. The external coil **14** may contain a receiver and transmitter antennae and a radio-frequency (RF) electronics card for communicating with the internal coil **116**.

Referring to **Figure 9**, a Fitting System (FS) may be used to configure and optimize the visual prosthesis apparatus shown in **Figure 16**. The Fitting System is fully described in the related application U.S. Application No. 11/796,425, filed on April 27, 2007, (Applicant's Docket No. S401-USA) which is incorporated herein by reference in its entirety.
The Fitting System may comprise custom software with a graphical user interface running on a dedicated laptop computer **10**. Within the Fitting System are modules for performing diagnostic checks of the implant, loading and executing video configuration files, viewing electrode voltage waveforms, and aiding in conducting psychophysical experiments. A video module can be used to download a video configuration file to the Video Processing Unit (VPU) **20** discussed above and store it in non-volatile memory to control various aspects of video configuration, e.g. the spatial relationship between the video input and the electrodes. The software can also load a previously used video configuration file from the VPU **20** for adjustment.

The Fitting System can be connected to the Psychophysical Test System (PTS), located for example on a dedicated laptop **30**, in order to run psychophysical experiments. In psychophysics mode, the Fitting System enables individual electrode control, permitting clinicians to construct test stimuli with control over current amplitude, pulse-width, and frequency of the stimulation. In addition, the psychophysics module allows the clinician to record subject responses. The PTS may include a collection of standard psychophysics experiments developed using for example MATLAB® (MathWorks)™ software and other tools to allow the clinicians to develop customized psychophysics experiment scripts.

Using the psychophysics module, important perceptual parameters such as perceptual threshold, maximum comfort level, and spatial location of percepts may be reliably measured. Based on these perceptual parameters, the fitting software enables custom configuration of the transformation between video image and spatio-temporal electrode stimulation parameters in an effort to optimize the effectiveness of the visual prosthesis for each subject.

The Fitting System laptop **10** of **Figure 9** may be connected to the VPU **20** using an optically isolated serial connection adapter **40**. Because it is optically isolated, the serial connection adapter **40** assures that no electric leakage current can flow from the Fitting System laptop **10** in the even of a fault condition.

As shown in **Figure 9**, the following components may be used with the Fitting System according to the present disclosure. The Video Processing Unit (VPU) **20** for the subject being tested, a Charged Battery **25** for VPU **20**, the Glasses **5**, a Fitting System (FS) Laptop **10**, a Psychophysical Test System (PTS) Laptop **30**, a PTS CD (not shown), a Communication Adapter (CA) **40**, a USB Drive (Security) (not shown), a USB Drive (Transfer) **47**, a USB Drive (Video Settings) (not shown), a Patient Input Device (RF Tablet) **50**, a further Patient Input Device (Jog Dial) **55**, Glasses Cable **15**, CA-VPU Cable **70**, FS-CA Cable **45**, FS-PTS Cable **46**, Four (4) Port USB Hub **47**, Mouse **60**, Test Array system **80**, Archival USB Drive **49**, an Isolation Transformer (not shown), adapter cables (not shown), and an External Monitor (not shown).

With continued reference to **Figure 9**, the external components of the Fitting System may be configured as follows. The battery **25** is connected with the VPU **20**. The PTS Laptop **30** is connected to FS Laptop **10** using the FS-PTS Cable **46**. The PTS Laptop **30** and FS Laptop **10** are plugged into the Isolation Transformer (not shown) using the Adapter Cables (not shown). The Isolation Transformer is plugged into the wall outlet. The four (4) Port USB Hub **47** is connected to the FS laptop **10** at the USB port. The mouse **60** and the two Patient Input Devices **50** and **55** are connected to four (4) Port USB Hubs **47**. The FS laptop **10** is connected to the Communication Adapter (CA) **40** using the FS-CA Cable **45**. The CA **40** is connected to the VPU **20** using the CA-VPU Cable **70**. The Glasses **5** are connected to the VPU **20** using the Glasses Cable **15**.

In one exemplary embodiment, the Fitting System shown in **Figure 9** may be used to configure system stimulation parameters and video processing strategies for each subject outfitted with the visual prosthesis apparatus of **Figure 1**. The fitting application, operating system, laptops **10** and **30**, isolation unit and VPU **20** may be tested and configuration controlled as a system. The software provides modules for electrode control, allowing an interactive construction of test stimuli with control over amplitude, pulse width, and frequency of the stimulation waveform of each electrode in the Retinal stimulation system 1. These parameters are checked to ensure that maximum charge per phase limits, charge balance, and power limitations are met before the test stimuli are presented to the subject. Additionally, these parameters may be checked a second time by the VPU **20's** firmware. The Fitting System shown in **Figure 9** may also provide a psychophysics module for administering a series of previously determined test stimuli to record subject's responses. These responses may be indicated by a keypad **50** and or verbally. The psychophysics module may also be used to reliably measure perceptual parameters such as perceptual threshold, maximum comfort level, and spatial location of percepts. These perceptual parameters may be used to custom configure the transformation between the video image and spatio-tempral electrode stimulation parameters thereby optimizing the effectiveness of the visual prosthesis for each subject. The Fitting System is fully described in the related application U.S. Application No. 11/796,425, filed on April 27, 2007, (Applicant's Docket No. S401-USA) which is incorporated herein by reference in its entirety.
The visual prosthesis apparatus may operate in two modes: i) stand-alone mode and ii) communication mode

### Stand-Alone Mode

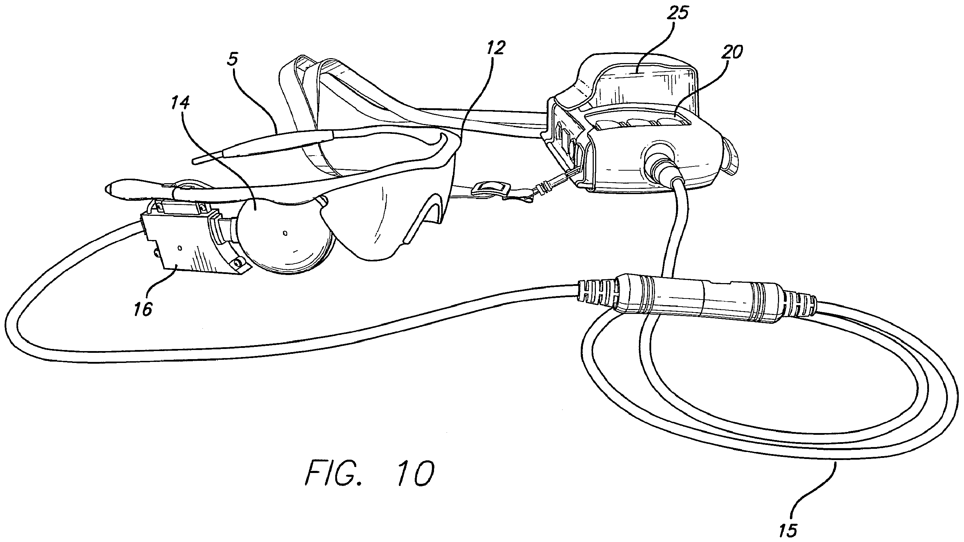

Referring to **Figure 10**, in the stand-alone mode, the video camera **12**, on the glasses **5**, captures a video image that is sent to the VPU **20**. The VPU **20** processes the image from the camera **12** and transforms it into electrical stimulation patterns that are transmitted to the external coil **14**. The external coil **14** sends the electrical stimulation patterns and power via radio-frequency (RF) telemetry to the implanted retinal stimulation system. The internal coil **116** of the retinal stimulation system **1** receives the RF commands from the external coil **14** and transmits them to the electronics package **4** that in turn delivers stimulation to the retina via the electrode array **2**. Additionally, the retinal stimulation system **1** may communicate safety and operational status back to the VPU **20** by transmitting RF telemetry from the internal coil **116** to the external coil **14**. The visual prosthesis apparatus of **Figure 1** may be configured to electrically activate the retinal stimulation system **1** only when it is powered by the VPU **20** through the external coil **14**. The stand-alone mode may be used for clinical testing and/or at-home use by the subject.

### Communication Mode

The communication mode may be used for diagnostic testing, psychophysical testing, patient fitting and downloading of stimulation settings to the VPU **20** before transmitting data from the VPU **20** to the retinal stimulation system **1** as is done for example in the stand-alone mode described above. Referring to **Figure 9**, in the communication mode, the VPU **20** is connected to the Fitting System laptop **10** using cables **70**, **45** and the optically isolated serial connection adapter **40**. In this mode, laptop **10** generated stimuli may be presented to the subject and programming parameters may be adjusted and downloaded to the VPU **20**. The Psychophysical Test System (PTS) laptop **30** connected to the Fitting System laptop **10** may also be utilized to perform more sophisticated testing and analysis as fully described in the related application U.S. Application No. 11/796,425, filed on April 27, 2007, (Applicant's Docket No. S401-USA) which is incorporated herein by reference in its entirety.

In one embodiment, the functionality of the retinal stimulation system can also be tested preoperatively and intra-operatively (i.e. before operation and during operation) by using an external coil **14**, without the glasses **5**, placed in close proximity to the retinal stimulation system **1**. The coil **14** may communicate the status of the retinal stimulation system **1** to the VPU **20** that is connected to the Fitting System laptop **10** as shown in **Figure 9**.

As discussed above, the VPU **20** processes the image from the camera **12** and transforms the image into electrical stimulation patterns for the retinal stimulation system. Filters such as edge detection filters may be applied to the electrical stimulation patterns for example by the VPU **20** to generate, for example, a stimulation pattern based on filtered video data that the VPU **20** turns into stimulation data for the retinal stimulation system. The images may then be reduced in resolution using a downscaling filter. In one exemplary embodiment, the resolution of the image may be reduced to match the number of electrodes in the electrode array **2010** of the retinal stimulation system. That is, if the electrode array has, for example, sixty electrodes, the image may be reduced to a sixty channel resolution. After the reduction in resolution, the image is mapped to stimulation intensity using for example a look-up table that has been derived from testing of individual subjects. Then, the VPU **20** transmits the stimulation parameters via forward telemetry to the retinal stimulation system in frames that may employ a cyclic redundancy check (CRC) error detection scheme.

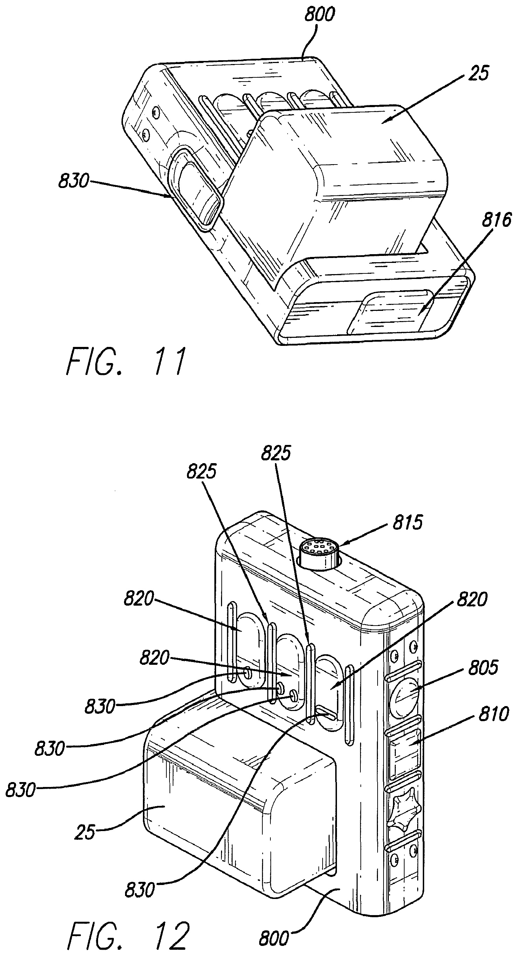

In one exemplary embodiment, the VPU **20** may be configured to allow the subject/patient i) to turn the visual prosthesis apparatus on and off, ii) to manually adjust settings, and iii) to provide power and data to the retinal stimulation system **1**. Referring to **Figures 11** and **12**, the VPU **20** may comprise a case **800**, power button **805** for turning the VPU **20** on and off, setting button **810**, zoom buttons **820** for controlling the camera **12**, connector port **815** for connecting to the Glasses **5**, a connector port **816** for connecting to the laptop **10** through the connection adapter **40**, indicator lights **825** to give visual indication of operating status of the system, the rechargeable battery **25** for powering the VPU **20**, battery latch **830** for locking the battery **25** in the case **800**, digital circuit boards (not shown), and a speaker (not shown) to provide audible alerts to indicate various operational conditions of the system. Because the VPU **20** is used and operated by a person with minimal or no vision, the buttons on the VPU **20** may be differently shaped and/or have special markings as shown in **Figure 12** to help the user identify the functionality of the button without having to look at it. As shown in Figure **12**, the power button **805** may be a circular shape while the settings button **820** may be square shape and the zoom buttons **820** may have special raised markings **830** to also identify each buttons functionality. One skilled in the art would appreciate that other shapes and markings can be used to identify the buttons without departing from the spirit and scope of the invention. For example, the markings can be recessed instead of raised.

In one embodiment, the indicator lights **825** may indicate that the VPU **20** is going through system start-up diagnostic testing when the one or more indicator lights **825** are blinking fast (more then once per second) and are green in color. The indicator lights **825** may indicate that the VPU **20** is operating normally when the one or more indicator lights **825** are blinking once per second and are green in color. The indicator lights **825** may indicate that the retinal stimulation system **1** has a problem that was detected by the VPU **20** at start-up diagnostic when the one or more indicator lights **825** are blinking for example once per five second and are green in color. The indicator lights **825** may indicate that the video signal from camera **12** is not being received by the VPU **20** when the one or more indicator lights **825** are always on and are amber color. The indicator lights **825** may indicate that there is a loss of communication between the retinal stimulation system **1** and the external coil **14** due to the movement or removal of Glasses **5** while the system is operational or if the VPU **20** detects a problem with the retinal stimulation system **1** and shuts off power to the retinal stimulation system **1** when the one or more indicator lights **825** are always on and are orange color. One skilled in the art would appreciate that other colors and blinking patterns can be used to give visual indication of operating status of the system without departing from the spirit and scope of the invention.

In one embodiment, a single short beep from the speaker (not shown) may be used to indicate that one of the buttons **825**, **805** or **810** have been pressed. A single beep followed by two more beeps from the speaker (not shown) may be used to indicate that VPU **20** is turned off. Two beeps from the speaker (not shown) may be used to indicate that VPU **20** is starting up. Three beeps from the speaker (not shown) may be used to indicate that an error has occurred and the VPU **20** is about to shut down automatically. As would be clear to one skilled in the are different periodic beeping may also be used to indicate a low battery voltage warning, that there is a problem with the video signal, and/or there is a loss of communication between the retinal stimulation system **1** and the external coil **14**. One skilled in the art would appreciate that other sounds can be used to give audio indication of operating status of the system without departing from the spirit and scope of the invention. For example, the beeps may be replaced by an actual prerecorded voice indicating operating status of the system.

In one exemplary embodiment, the VPU **20** is in constant communication with the retinal stimulation system through forward and backward telemetry. In this document, the forward telemetry refers to transmission from VPU **20** to the retinal stimulation system **1** and the backward telemetry refers to transmissions from the Retinal stimulation system **1** to the VPU **20**. During the initial setup, the VPU **20** may transmit null frames (containing no stimulation information) until the VPU **20** synchronizes with the Retinal stimulation system **1** via the back telemetry. In one embodiment, an audio alarm may be used to indicate whenever the synchronization has been lost.

In order to supply power and data to the Retinal stimulation system **1**, the VPU **20** may drive the external coil **14**, for example, with a 3 MHz signal. To protect the subject, the retinal stimulation system **1** may comprise a failure detection circuit to detect direct current leakage and to notify the VPU **20** through back telemetry so that the visual prosthesis apparatus can be shut down.

The forward telemetry data (transmitted for example at 122.76 kHz) may be modulated onto the exemplary 3 MHz carrier using Amplitude Shift Keying (ASK), while the back telemetry data (transmitted for example at 3.8 kHz) may be modulated using Frequency Shift Keying (FSK) with, for example, 442 kHz and 457 kHz. The theoretical bit error rates can be calculated for both the ASK and FSK scheme assuming a ratio of signal to noise (SNR). The system disclosed in the present disclosure can be reasonably expected to see bit error rates of 10-5 on forward telemetry and 10-3 on back telemetry. These errors may be caught more than 99.998% of the time by both an ASIC hardware telemetry error detection algorithm and the VPU **20's** firmware. For the forward telemetry, this is due to the fact that a 16-bit cyclic redundancy check (CRC) is calculated for every 1024 bits sent to the ASIC within electronics package **4** of the Retinal Stimulation System **1**. The ASIC of the Retinal Stimulation System **1** verifies this CRC and handles corrupt data by entering a non-stimulating 'safe' state and reporting that a telemetry error was detected to the VPU **20** via back telemetry. During the 'safe' mode, the VPU **20** may attempt to return the implant to an operating state. This recovery may be on the order of milliseconds. The back telemetry words are checked for a 16-bit header and a single parity bit. For further protection against corrupt data being misread, the back telemetry is only checked for header and parity if it is recognized as properly encoded Bi-phase Mark Encoded (BPM) data. If the VPU **20** detects invalid back telemetry data, the VPU **20** immediately changes mode to a 'safe' mode where the Retinal Stimulation System **1** is reset and the VPU **20** only sends non-stimulating data frames. Back telemetry errors cannot cause the VPU **20** to do anything that would be unsafe.

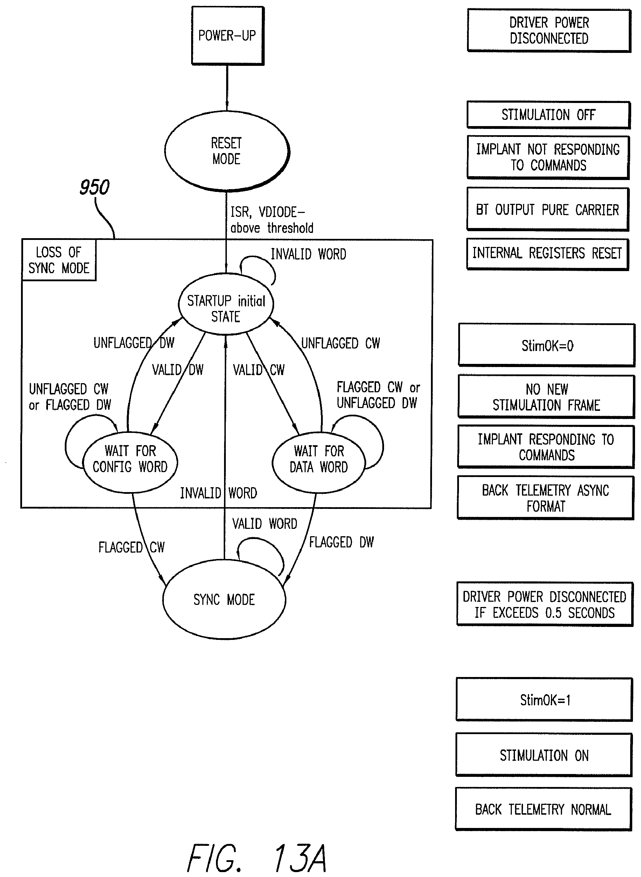

The response to errors detected in data transmitted by VPU **20** may begin at the ASIC of the Retinal Stimulation System **1**. The Retinal Stimulation System **1** may be constantly checking the headers and CRCs of incoming data frames. If either the header or CRC check fails, the ASIC of the Retinal Stimulation System **1** may enter a mode called LOSS OF SYNC **950**, shown in **Figure 13a**. In LOSS OF SYNC mode **950**, the Retinal Stimulation System **1** will no longer produce a stimulation output, even if commanded to do so by the VPU **20**. This cessation of stimulation occurs after the end of the stimulation frame in which the LOSS OF SYNC mode **950** is entered, thus avoiding the possibility of unbalanced pulses not completing stimulation. If the Retinal Stimulation System **1** remains in a LOSS OF SYNC mode **950** for 1 second or more (for example, caused by successive errors in data transmitted by VPU **20**), the ASIC of the Retinal Stimulation System **1** disconnects the power lines to the stimulation pulse drivers. This eliminates the possibility of any leakage from the power supply in a prolonged LOSS OF SYNC mode **950**. From the LOSS OF SYNC mode **950**, the Retinal Stimulation System **1** will not reenter a stimulating mode until it has been properly initialized with valid data transmitted by the VPU **20**.

In addition, the VPU **20** may also take action when notified of the LOSS OF SYNC mode **950**. As soon as the Retinal Stimulation System **1** enters the LOSS OF SYNC mode **950**, the Retinal Stimulation System **1** reports this fact to the VPU **20** through back telemetry. When the VPU **20** detects that the Retinal Stimulation System **1** is in LOSS OF SYNC mode **950**, the VPU **20** may start to send 'safe' data frames to the Retinal Stimulation System **1**. 'Safe' data is data in which no stimulation output is programmed and the power to the stimulation drivers is also programmed to be off. The VPU **20** will not send data frames to the Retinal Stimulation System **1** with stimulation commands until the VPU **20** first receives back telemetry from the Retinal Stimulation System **1** indicating that the Retinal Stimulation System **1** has exited the LOSS OF SYNC mode **950**. After several unsuccessful retries by the VPU **20** to take the implant out of LOSS OF SYNC mode **950**, the VPU **20** will enter a Low Power Mode (described below) in which the implant is only powered for a very short time. In this time, the VPU **20** checks the status of the implant. If the implant continues to report a LOSS OF SYNC mode **950**, the VPU **20** turns power off to the Retinal Stimulation System **1** and tries again later. Since there is no possibility of the implant electronics causing damage when it is not powered, this mode is considered very safe.

Due to an unwanted electromagnetic interference (EMI) or electrostatic discharge (ESD) event the VPU **20** data, specifically the VPU firmware code, in RAM can potentially get corrupted and may cause the VPU **20** firmware to freeze. As a result, the VPU **20** firmware will stop resetting the hardware watchdog circuit, which may cause the system to reset. This will cause the watchdog timer to expire causing a system reset in, for example, less than 2.25 seconds. Upon recovering from the reset, the VPU **20** firmware logs the event and shuts itself down. VPU **20** will not allow system usage after this occurs once. This prevents the VPU **20** code from freezing for extended periods of time and hence reduces the probability of the VPU sending invalid data frames to the implant.

Supplying power to the Retinal stimulation system **1** can be a significant portion of the VPU **20's** total power consumption. When the Retinal stimulation system **1** is not within receiving range to receive either power or data from the VPU **20**, the power used by the VPU **20** is wasted.

Power delivered to the Retinal stimulation system **1** may be dependant on the orientation of the coils **14** and **116**. The power delivered to the Retinal stimulation system **1** may be controlled, for example, via the VPU **20** every 16.6 ms. The Retinal stimulation system **1** may report how much power it receives and the VPU **20** may adjust the power supply voltage of the RF driver to maintain a required power level on the Retinal stimulation system **1**. Two types of power loss may occur: 1) long term (> ∼ 1 second) and 2) short term (< ∼ 1 second). The long term power loss may be caused, for example, by a subject removing the Glasses **5**.

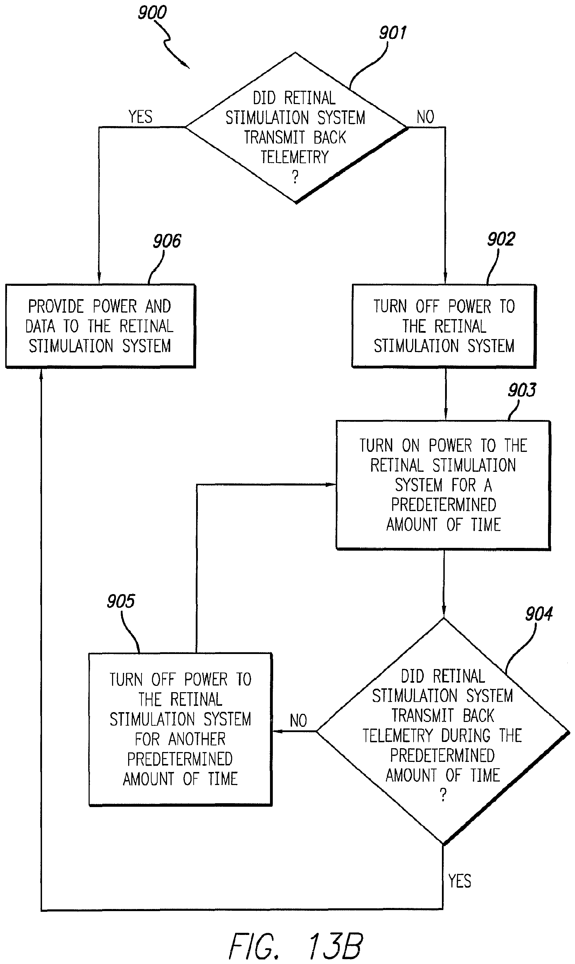

In one exemplary embodiment, the Low Power Mode may be implemented to save power for VPU **20**. The Low Power Mode may be entered, for example, anytime the VPU **20** does not receive back telemetry from the Retinal stimulation system **1**. Upon entry to the Low Power Mode, the VPU **20** turns off power to the Retinal stimulation system **1**. After that, and periodically, the VPU **20** turns power back on to the Retinal stimulation system **1** for an amount of time just long enough for the presence of the Retinal stimulation system **1** to be recognized via its back telemetry. If the Retinal stimulation system **1** is not immediately recognized, the controller again shuts off power to the Retinal stimulation system **1**. In this way, the controller 'polls' for the passive Retinal stimulation system **1** and a significant reduction in power used is seen when the Retinal stimulation system **1** is too far away from its controller device. **Figure 13b** depicts an exemplary block diagram **900** of the steps taken when the VPU **20** does not receive back telemetry from the Retinal stimulation system **1.** If the VPU **20** receives back telemetry from the Retinal stimulation system **1** (output "YES" of step **901**), the Retinal stimulation system **1** may be provided with power and data (step **906**). If the VPU **20** does not receive back telemetry from the Retinal stimulation system **1** (output "NO" of step **901**), the power to the Retinal stimulation system **1** may be turned off. After some amount of time, power to the Retinal stimulation system **1** may be turned on again for enough time to determine if the Retinal stimulation system **1** is again transmitting back telemetry (step **903**). If the Retinal stimulation system **1** is again transmitting back telemetry (step **904**), the Retinal stimulation system **1** is provided with power and data (step **906**). If the Retinal stimulation system **1** is not transmitting back telemetry (step **904**), the power to the Retinal stimulation system **1** may again be turned off for a predetermined amount of time (step **905**) and the process may be repeated until the Retinal stimulation system **1** is again transmitting back telemetry.

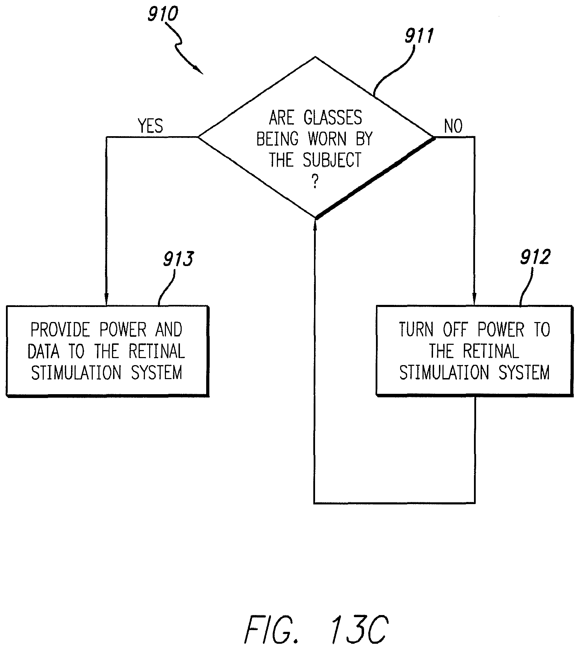

In another exemplary embodiment, the Low Power Mode may be entered whenever the subject is not wearing the Glasses **5**. In one example, the Glasses **5** may contain a capacitive touch sensor (not shown) to provide the VPU **20** digital information regarding whether or not the Glasses **5** are being worn by the subject. In this example, the Low Power Mode may be entered whenever the capacitive touch sensor detects that the subject is not wearing the Glasses **5**. That is, if the subject removes the Glasses **5**, the VPU **20** will shut off power to the external coil **14**. As soon as the Glasses **5** are put back on, the VPU **20** will resume powering the external coil **14**. **Figure 13c** depicts an exemplary block diagram **910** of the steps taken when the capacitive touch sensor detects that the subject is not wearing the Glasses **5.** If the subject is wearing Glasses **5** (step **911**), the Retinal stimulation system **1** is provided with power and data (step **913**). If the subject is not wearing Glasses **5** (step **911**), the power to the Retinal stimulation system **1** is turned off (step **912**) and the process is repeated until the subject is wearing Glasses **5**.

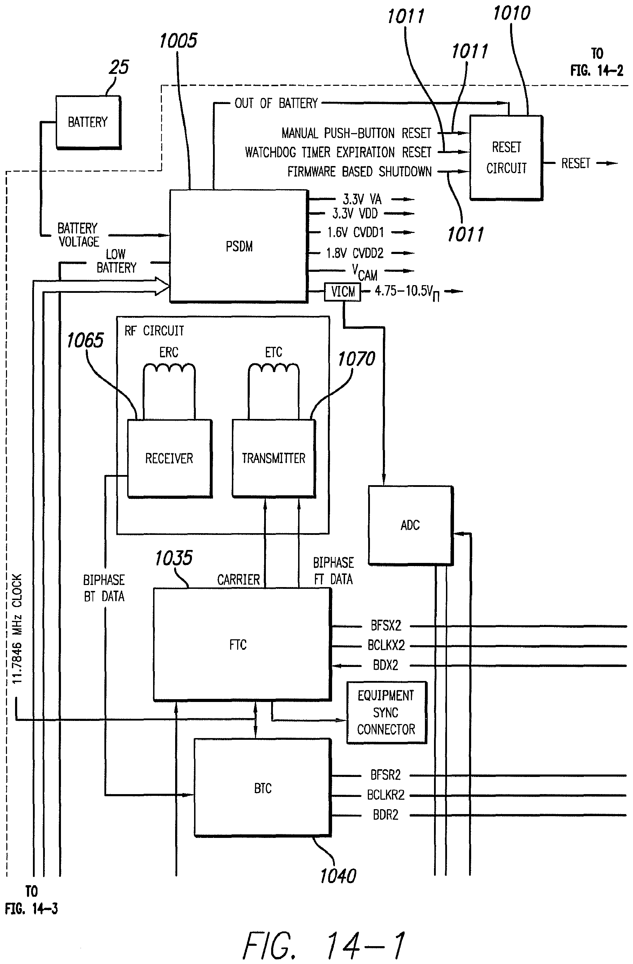

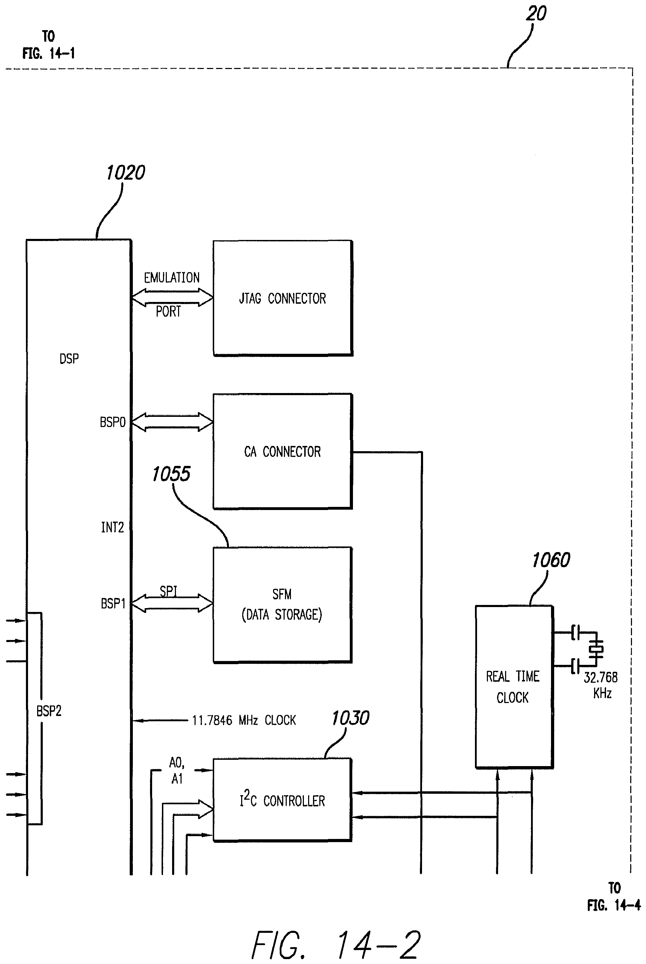

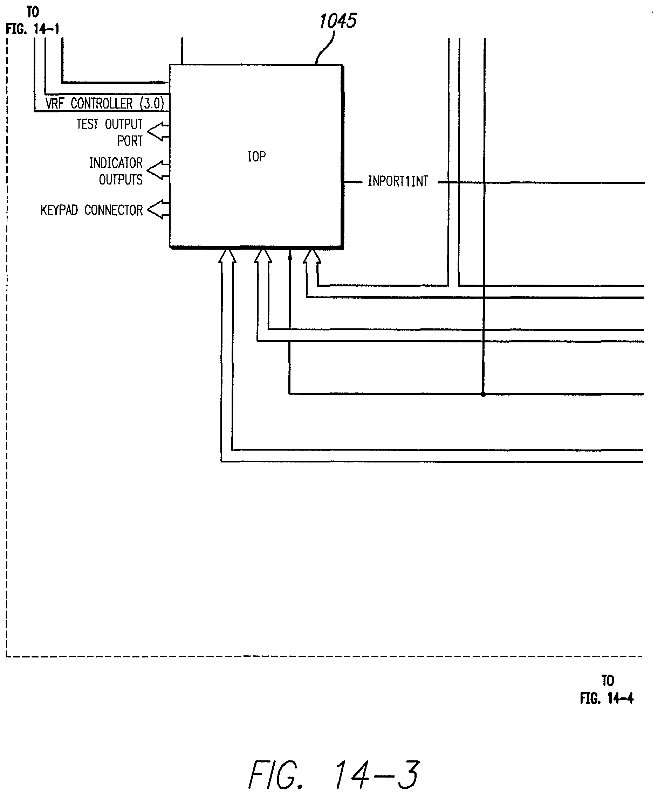

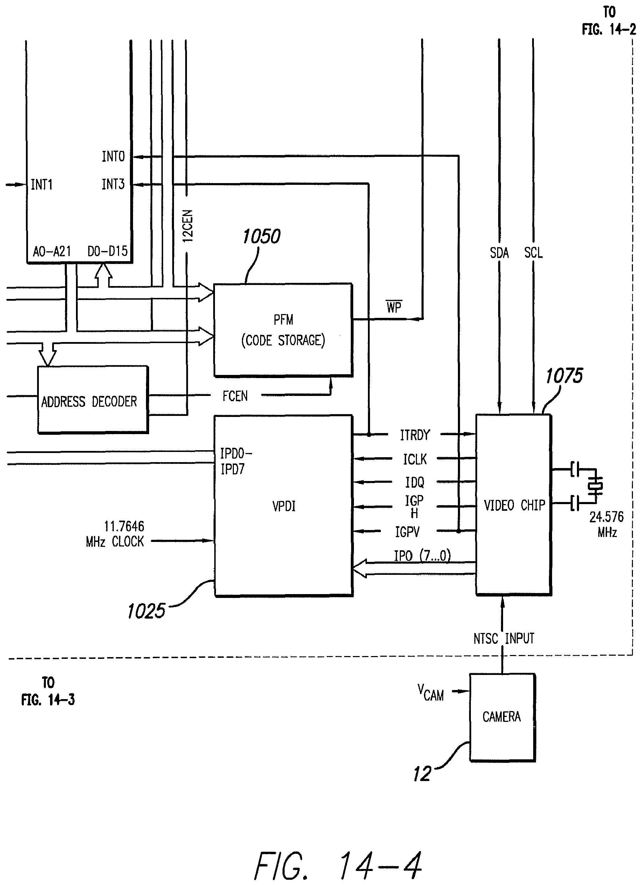

One exemplary embodiment of the VPU **20** is shown in **Figure 14**. The VPU **20** may comprise: a Power Supply, a Distribution and Monitoring Circuit (PSDM) **1005**, a Reset Circuit **1010**, a System Main Clock (SMC) source (not shown), a Video Preprocessor Clock (VPC) source (not shown), a Digital Signal Processor (DSP) **1020**, Video Preprocessor Data Interface **1025**, a Video Preprocessor **1075**, an I²C Protocol Controller **1030**, a Complex Programmable Logic device (CPLD) (not shown), a Forward Telemetry Controller (FTC) **1035**, a Back Telemetry Controller (BTC) **1040**, Input/Output Ports **1045**, Memory Devices like a Parallel Flash Memory (PFM) **1050** and a Serial Flash Memory (SFM) **1055**, a Real Time Clock **1060**, an RF Voltage and Current Monitoring Circuit (VIMC) (not shown), a speaker and/or a buzzer, an RF receiver **1065**, and an RF transmitter **1070**.

The Power Supply, Distribution and Monitoring Circuit (PSDM) **1005** may regulate a variable battery voltage to several stable voltages that apply to components of the VPU **20**. The Power Supply, Distribution and Monitoring Circuit (PSDM) **1005** may also provide low battery monitoring and depleted battery system cutoff. The Reset Circuit **1010** may have reset inputs **1011** that are able to invoke system level rest. For example, the reset inputs **1011** may be from a manual push-button reset, a watchdog timer expiration, and/or firmware based shutdown. The System Main Clock (SMC) source is a clock source for DSP **1020** and CPLD. The Video Preprocessor Clock (VPC) source is a clock source for the Video Processor. The DSP **1020** may act as the central processing unit of the VPU **20**. The DSP **1020** may communicate with the rest of the components of the VPU **20** through parallel and serial interfaces. The Video Processor **1075** may convert the NTSC signal from the camera **12** into a down-scaled resolution digital image format. The Video Processor **1075** may comprise a video decoder (not shown) for converting the NTSC signal into high-resolution digitized image and a video scaler (not shown) for scaling down the high-resolution digitized image from the video decoder to an intermediate digitized image resolution. The video decoder may be composed of an Analog Input Processing, Chrominance and Luminance Processing and Brightness Contrast and Saturation (BSC) Control circuits. The video scaler may be composed of Acquisition control, Pre-scaler, BSC-control, Line Buffer and Output Interface. The I²C Protocol Controller **1030** may serve as a link between the DSP **1020** and the I²C bus. The I²C Protocol Controller **1030** may be able to convert the parallel bus interface of the DSP **1020** to the I²C protocol bus or vise versa. The I²C Protocol Controller **1030** may also be connected to the Video Processor **1075** and the Real Time Clock **1060**. The VPDI **1025** may contain a tri-state machine to shift video data from Video Preprocessor **1075** to the DSP **1020**. The Forward Telemetry Controller (FTC) **1035** packs 1024 bits of forward telemetry data into a forward telemetry frame. The FTC **1035** retrieves the forward telemetry data from the DSP **1020** and converts the data from logic level to biphase marked data. The Back Telemetry Controller (BTC) **1040** retrieves the biphase marked data from the RF receiver **1065**, decodes it, and generates the BFSR and BCLKR for the DSP **1020**. The Input/Output Ports **1045** provide expanded IO functions to access the CPLD on-chip and off-chip devices. The Parallel Flash Memory (PFM) **1050** may be used to store executable code and the Serial Flash Memory (SFM) **1055** may provide Serial Port Interface (SPI) for data storage. The VIMC may be used to sample and monitor RF transmitter **1070** current and voltage in order to monitor the integrity status of the retinal stimulation system **1**.

**Figure 16** shows a perspective view of the implanted portion of the preferred visual prosthesis. A flexible circuit **2001** includes a flexible circuit electrode array **2010** which is mounted by a retinal tack (not shown) or similar means to the epiretinal surface. The flexible circuit electrode array **2010** is electrically coupled by a flexible circuit cable **2012**, which pierces the sclera and is electrically coupled to an electronics package **2014**, external to the sclera.

The electronics package **2014** is electrically coupled to a secondary inductive coil **2016**. Preferably the secondary inductive coil **2016** is made from wound wire. Alternatively, the secondary inductive coil **2016** may be made from a flexible circuit polymer sandwich with wire traces deposited between layers of flexible circuit polymer. The secondary inductive coil receives power and data from a primary inductive coil **2017**, which is external to the body. The electronics package **2014** and secondary inductive coil **2016** are held together by the molded body **2018**. The molded body **18** holds the electronics package **2014** and secondary inductive coil **16** end to end. The secondary inductive coil **16** is placed around the electronics package **2014** in the molded body **2018**. The molded body **2018** holds the secondary inductive coil **2016** and electronics package **2014** in the end to end orientation and minimizes the thickness or height above the sclera of the entire device. The molded body **2018** may also include suture tabs **2020**. The molded body **2018** narrows to form a strap **2022** which surrounds the sclera and holds the molded body **2018**, secondary inductive coil **2016**, and electronics package **2014** in place. The molded body **2018**, suture tabs **2020** and strap **2022** are preferably an integrated unit made of silicone elastomer. Silicone elastomer can be formed in a pre-curved shape to match the curvature of a typical sclera. However, silicone remains flexible enough to accommodate implantation and to adapt to variations in the curvature of an individual sclera. The secondary inductive coil **2016** and molded body **2018** are preferably oval shaped. A strap **2022** can better support an oval shaped coil. It should be noted that the entire implant is attached to and supported by the sclera. An eye moves constantly. The eye moves to scan a scene and also has a jitter motion to improve acuity. Even though such motion is useless in the blind, it often continues long after a person has lost their sight. By placing the device under the rectus muscles with the electronics package in an area of fatty tissue between the rectus muscles, eye motion does not cause any flexing which might fatigue, and eventually damage, the device.

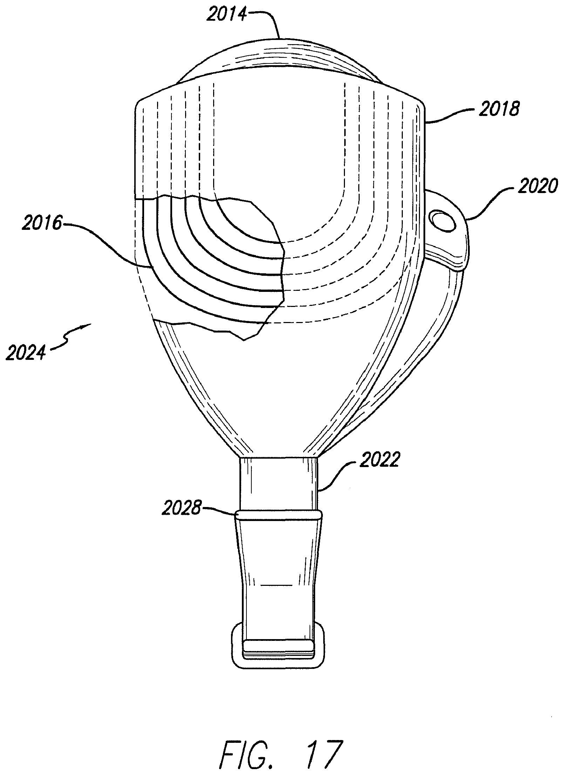

**Figure 17** shows a side view of the implanted portion of the visual prosthesis, in particular, emphasizing the fan tail **2024**. When implanting the visual prosthesis, it is necessary to pass the strap **2022** under the eye muscles to surround the sclera. The secondary inductive coil **2016** and molded body **2018** must also follow the strap **2022** under the lateral rectus muscle on the side of the sclera. The implanted portion of the visual prosthesis is very delicate. It is easy to tear the molded body **2018** or break wires in the secondary inductive coil **2016**. In order to allow the molded body **18** to slide smoothly under the lateral rectus muscle, the molded body **2018** is shaped in the form of a fan tail **2024** on the end opposite the electronics package **2014.** The strap **2022** further includes a hook **2028** the aids the surgeon in passing the strap under the rectus muscles.

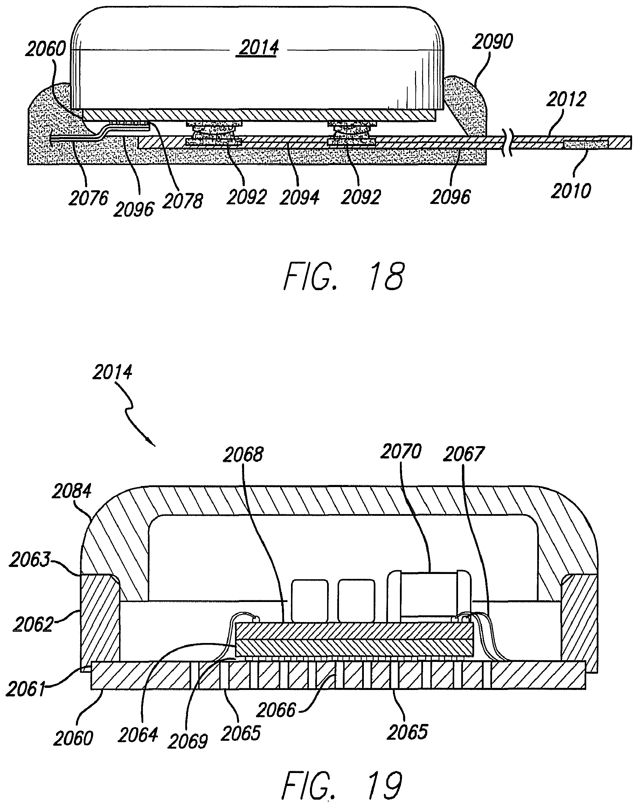

Referring to **figure 18**, the flexible circuit **1**, includes platinum conductors **2094** insulated from each other and the external environment by a biocompatible dielectric polymer **2096**, preferably polyimide. One end of the array contains exposed electrode sites that are placed in close proximity to the retinal surface **2010**. The other end contains bond pads **2092** that permit electrical connection to the electronics package **2014**. The electronic package **2014** is attached to the flexible circuit **1** using a flip-chip bumping process, and epoxy underfilled. In the flip-chip bumping process, bumps containing conductive adhesive placed on bond pads **2092** and bumps containing conductive adhesive placed on the electronic package **2014** are aligned and melted to build a conductive connection between the bond pads **2092** and the electronic package **2014**. Leads **2076** for the secondary inductive coil **2016** are attached to gold pads **2078** on the ceramic substrate **2060** using thermal compression bonding, and are then covered in epoxy. The electrode array cable **2012** is laser welded to the assembly junction and underfilled with epoxy. The junction of the secondary inductive coil **2016**, array **2001**, and electronic package **2014** are encapsulated with a silicone overmold **2090** that connects them together mechanically. When assembled, the hermetic electronics package **2014** sits about 3mm away from the end of the secondary inductive coil.

Since the implant device is implanted just under the conjunctiva it is possible to irritate or even erode through the conjunctiva. Eroding through the conjunctiva leaves the body open to infection. We can do several things to lessen the likelihood of conjunctiva irritation or erosion. First, it is important to keep the over all thickness of the implant to a minimum. Even though it is advantageous to mount both the electronics package **2014** and the secondary inductive coil **2016** on the lateral side of the sclera, the electronics package **2014** is mounted higher than, but not covering, the secondary inductive coil **2016**. In other words the thickness of the secondary inductive coil **2016** and electronics package should not be cumulative.

It is also advantageous to place protective material between the implant device and the conjunctiva. This is particularly important at the scleratomy, where the thin film electrode array cable **2012** penetrates the sclera. The thin film electrode array cable **2012** must penetrate the sclera through the pars plana, not the retina. The scleratomy is, therefore, the point where the device comes closest to the conjunctiva. The protective material can be provided as a flap attached to the implant device or a separate piece placed by the surgeon at the time of implantation. Further material over the scleratomy will promote healing and sealing of the scleratomy. Suitable materials include DACRON®, TEFLON®, GORETEX® (ePTFE), TUTOPLAST® (sterilized sclera), MERSILENE® (polyester) or silicone.

Referring to **figure 19**, the package **2014** contains a ceramic substrate **2060**, with metalized vias **2065** and thin-film metallization **2066**. The package **2014** contains a metal case wall **2062** which is connected to the ceramic substrate **2060** by braze joint **2061**. On the ceramic substrate **2060** an underfill **2069** is applied. On the underfill **69** an integrated circuit chip **2064** is positioned. On the integrated circuit chip **2064** a ceramic hybrid substrate **2068** is positioned. On the ceramic hybrid substrate **2068** passives **2070** are placed. Wirebonds **2067** are leading from the ceramic substrate **2060** to the ceramic hybrid substrate **2068**. A metal lid **2084** is connected to the metal case wall **2062** by laser welded joint **2063** whereby the package **2014** is sealed.
Accordingly, what has been shown is an improved visual prosthesis and an improved method for limiting power consumption in a visual prosthesis. While the invention has been described by means of specific embodiments and applications thereof, it is understood that numerous modifications and variations could be made thereto by those skilled in the art without departing from the scope of the invention as defined in the appended claims. It is therefore to be understood that within the scope of the claims, the invention may be practiced otherwise than as specifically described herein.

## Claims

1. A visual prosthesis system for fitting, training or assessing performance comprising:
a visual prosthesis;
a touch sensitive display configured to display an image;
a computer connected to the touch sensitive display and the visual prosthesis, the computer configured to record touch data from the touch sensitive display of a user of the visual prosthesis touching the display indicating a direction of motion of the perceived image or a location of the perceived image; and
the computer configured to adjust the visual prosthesis based on the image and the touch data.

2. The visual prosthesis system of claim 1, wherein the computer is configured to adjust rotation, translation or skew.

3. The visual prosthesis system of claim 1, wherein the computer is configured to adjust by selecting a subset of data from an image capture device.

4. The visual prosthesis system of claim 1, wherein the computer is configured to adjust by physically adjusting an image capture device.

5. The visual prosthesis system of claim 4, wherein physically adjusting is translation or rotation of the image capture device.

6. The visual prosthesis system of claim 1, wherein a target within the image is moving and the touch sensitive display records a movement of touch.

7. The visual prosthesis system of claim 6, wherein the target has a higher intensity at its center and a lower intensity at its edges.

8. The visual prosthesis system of claim 1, wherein a target within the image is stationary and the touch sensitive display is configured to record a location of touch.

9. The visual prosthesis system of claim 8, wherein the target has a larger dimension perpendicular to its direction of motion.

10. The visual prosthesis system of claim 1, wherein adjusting is based upon moment analysis, cluster analysis and/or non-Gaussian analysis.

11. The visual prosthesis system of claim 1, wherein the visual prosthesis includes an image capture device on a flexible mount providing for translation and rotation of the image capture device relative to a user's head.

12. The visual prosthesis system of claim 1, further comprising a video memory device including computer for selecting a subset of video memory for presentation to a subject.

13. The visual prosthesis system of claim 1, further comprising a target within the touch sensitive display which is brighter at its center and less bright at its edges.

## Patentansprüche

1. Sichtprothesesystem zum Anpassen, Trainieren oder Auswerten einer Leistung, das Folgendes umfasst:
eine Sichtprothese;
eine berührungsempfindliche Anzeige, die ausgelegt ist, um ein Bild anzuzeigen;
einen Computer, der mit der berührungsempfindlichen Anzeige und der Sichtprothese verbunden ist, wobei der Computer konfiguriert ist, um Berührungsdaten von der berührungsempfindlichen Anzeige eines Benutzers der Sichtprothese, der die Anzeige berührt, wobei eine Bewegungsrichtung des wahrgenommenen Bilds oder eine Position des wahrgenommenen Bilds angegeben wird, aufzuzeichnen; und
wobei der Computer konfiguriert ist, die Sichtprothese basierend auf dem Bild und den Berührungsdaten einzustellen.

2. Sichtprothesesystem nach Anspruch 1, wobei der Computer konfiguriert ist, um Drehung, Verschiebung oder Schräglage einzustellen.

3. Sichtprothesesystem nach Anspruch 1, wobei der Computer konfiguriert ist, um durch Auswählen eines Teilsatzes von Daten von einer Bildaufnahmevorrichtung Einstellungen vorzunehmen.

4. Sichtprothesesystem nach Anspruch 1, wobei der Computer konfiguriert ist, um durch physisches Einstellen einer Bildaufnahmevorrichtung Einstellungen vorzunehmen.

5. Sichtprothesesystem nach Anspruch 4, wobei das physische Einstellen ein Verschieben oder ein Drehen der Bildaufnahmevorrichtung ist.

6. Sichtprothesesystem nach Anspruch 1, wobei sich ein Ziel innerhalb des Bilds bewegt und die berührungsempfindliche Anzeige eine Bewegung einer Berührung aufzeichnet.

7. Sichtprothesesystem nach Anspruch 6, wobei das Ziel in seiner Mitte eine höhere Intensität und an seinen Rändern eine niedrigere Intensität aufweist.

8. Sichtprothesesystem nach Anspruch 1, wobei ein Ziel innerhalb des Bilds unbewegt ist und die berührungsempfindliche Anzeige ausgelegt ist, um eine Berührungsstelle aufzuzeichnen.

9. Sichtprothesesystem nach Anspruch 8, wobei das Ziel normal auf seine Bewegungsrichtung eine größere Abmessung aufweist.

10. Sichtprothesesystem nach Anspruch 1, wobei das Einstellen auf einer Momentanalyse, Clusteranalyse und/oder einer nichtgaußschen Analyse basiert.

11. Sichtprothesesystem nach Anspruch 1, wobei die Sichtprothese eine Bildaufnahmevorrichtung auf einer flexiblen Halterung umfasst, die eine Verschiebung und Drehung der Bildaufnahmevorrichtung relativ zum Kopf eines Benutzers bereitstellt.

12. Sichtprothesesystem nach Anspruch 1, das ferner einen eine Videospeichervorrichtung umfassenden Computer zum Auswählen eines Teilsatzes des Videospeichers zur Darstellung an ein Individuum aufweist.

13. Sichtprothesesystem nach Anspruch 1, das ferner ein Ziel innerhalb der berührungsempfindlichen Anzeige umfasst, das in seiner Mitte heller ist und an seinen Rändern weniger hell ist.

## Revendications

1. Système de prothèse visuelle pour un ajustement, un apprentissage ou une évaluation de performance comprenant :
une prothèse visuelle ;
un écran tactile configuré pour afficher une image ;
un ordinateur connecté à l'écran tactile et à la prothèse visuelle, l'ordinateur étant configuré pour enregistrer des données tactiles à partir de l'écran tactile d'un utilisateur de la prothèse visuelle touchant l'écran en indiquant une direction de mouvement de l'image perçue ou un emplacement de l'image perçue ; et
l'ordinateur configuré pour ajuster la prothèse visuelle en fonction de l'image et des données tactiles.

2. Système de prothèse visuelle selon la revendication 1, dans lequel l'ordinateur est configuré pour ajuster une rotation, une translation ou une inclinaison.

3. Système de prothèse visuelle selon la revendication 1, dans lequel l'ordinateur est configuré pour ajuster en sélectionnant un sous-ensemble de données à partir d'un dispositif de capture d'image.

4. Système de prothèse visuelle selon la revendication 1, dans lequel l'ordinateur est configuré pour ajuster en ajustant physiquement un dispositif de capture d'image.

5. Système de prothèse visuelle selon la revendication 4, dans lequel l'ajustement physique est une translation ou une rotation du dispositif de capture d'image.

6. Système de prothèse visuelle selon la revendication 1, dans lequel une cible dans l'image se déplace et l'écran tactile enregistre un mouvement de toucher.

7. Système de prothèse visuelle selon la revendication 6, dans lequel la cible a une intensité plus élevée en son centre et une intensité inférieure au niveau de ses bords.

8. Système de prothèse visuelle selon la revendication 1, dans lequel une cible dans l'image est stationnaire et l'écran tactile est configuré pour enregistrer un emplacement de toucher.

9. Système de prothèse visuelle selon la revendication 8, dans lequel la cible a une plus grande dimension perpendiculaire à sa direction de mouvement.

10. Système de prothèse visuelle selon la revendication 1, dans lequel l'ajustement est basé sur une analyse de moment, une analyse d'agrégats et/ou une analyse non gaussienne.

11. Système de prothèse visuelle selon la revendication 1, dans lequel la prothèse visuelle comprend un dispositif de capture d'image sur un support flexible assurant une translation et une rotation du dispositif de capture d'image par rapport à la tête d'un utilisateur.

12. Système de prothèse visuelle selon la revendication 1, comprenant en outre un dispositif de mémoire vidéo comprenant un ordinateur pour sélectionner un sous-ensemble de mémoire vidéo pour une présentation à un sujet.

13. Système de prothèse visuelle selon la revendication 1, comprenant en outre une cible à l'intérieur de l'écran tactile qui est plus lumineuse en son centre et moins lumineuse au niveau de ses bords.
